# EUROPEAN PATENT APPLICATION

(11) **EP 4 407 038 A1**
(43) Date of publication of application: **31.07.2024**
(21) Application number: 22872052.0
(22) Date of filing: 22.09.2022
(51) Int. Cl.: C12P 19/04, C12P 19/18, C12N 1/21, C12N 1/32

(54) **METHOD FOR PREPARING STARCH USING CARBON DIOXIDE, RECOMBINANT MICROORGANISM, AND METHOD FOR CONSTRUCTING RECOMBINANT MICROORGANISM**

(30) Priority: 23.09.2021 CN 202111116011; 19.09.2022 CN 202211137776
(71) Applicant: Tianjin Institute of Industrial Biotechnology, Chinese Academy of Sciences, Tianjin Airport Economic Area Tianjin 300308 (CN)
(72) Inventor: MA, Yanhe, Tianjin 300308 (CN); CAI, Tao, Tianjin 300308 (CN); SUN, Hongbing, Tianjin 300308 (CN); WANG, Qinhong, Tianjin 300308 (CN); WANG, Guokun, Tianjin 300308 (CN); XU, Zhaoyu, Tianjin 300308 (CN); ZHU, Zhiguang, Tianjin 300308 (CN); MA, Chunling, Tianjin 300308 (CN); ZHENG, Ping, Tianjin 300308 (CN); WANG, Yu, Tianjin 300308 (CN); QIAO, Jing, Tianjin 300308 (CN); DONG, Hongjun, Tianjin 300308 (CN); GUO, Wei, Tianjin 300308 (CN); ZHOU, Hongyi, Tianjin 300308 (CN)
(74) Representative: Cabinet Bleger-Rhein-Poupon
(86) International application number: PCT/CN2022/120499
(87) International publication number: WO 2023/046007

(57) **Abstract**

Provided are a method for preparing starch using carbon dioxide, a recombinant microorganism, a method for constructing the recombinant microorganism, and a reagent. The method for preparing starch using carbon dioxide comprises: (1) providing energy and carbon sources for microbial cells on the basis of carbon dioxide and extracellular non-optical energy; and (2) generating starch within the microbial cells on the basis of at least one of up-regulated glucose-1-phosphate adenylyltransferase and starch synthase in the microbial cells. In this way, by utilizing non-optical energy, such as electric energy or hydrogen energy, starch can be effectively prepared inside the microbial cells by fixing carbon dioxide.

## Description

### Technical Field

The present application belongs to the field of biotechnology, and specifically relates to a method for preparing starch using carbon dioxide, a recombinant microorganism, and a method for constructing the recombinant microorganism.

### Background

Starch is the most important nutrient in grain. It provides more than 80% of the calories in the world. Among the nearly 2 billion tons of cereal grains produced worldwide every year, 1.2-1.4 billion tons are starch. At present, agricultural planting is still the only way to produce starch. Global food production needs to consume 38% of land and 70% of fresh water resources, about 200 million tons of chemical fertilizers and 3 million tons of pesticides every year. With the increase of global population, it is predicted that the global food demand will increase by more than 70% by 2050. It is difficult for the current agricultural planting methods to meet the growing food demand, and it will be of great significance to develop methods that can replace agricultural planting to produce starch.

Excessive emission of carbon dioxide leads to global warming, acidification of seawater, and irreversible damage to the environment. Since the industrial revolution, the concentration of carbon dioxide in the atmosphere has increased from 277 ppm to 400 ppm, which is equivalent to a net increase of 1 trillion tons at a rate of 3 ppm every year. Photosynthetic organisms such as plants utilize optical energy to convert and fix about 100 billion tons of carbon dioxide every year, so the carbon cycle system of the earth can't digest so much carbon dioxide. Rapid bio-transformation of carbon dioxide can be realized only by breaking through the limit of natural photosynthesis, creating a more efficient artificial photosystem, and greatly improving the efficiency of biological carbon fixation.

Therefore, how to use carbon dioxide scientifically to help carbon-neutral strategies and how to realize biological industrial synthesis of starch so as to realize "agricultural industrialization" are difficult problems to be solved urgently.

Microalgae and cyanobacteria in photosynthetic organisms can grow directly using carbon dioxide fixed by optical energy, and become excellent chassis cells for developing carbon fixation by cells due to their advantages such as fast growth speed and high energy utilization efficiency. At present, related articles and patents have reported cases of starch production by algae (Improving carbohydrate and starch accumulation in Chlorella sp. AE10 by a novel two-stage process with cell dilution, 2017; JP07059557A, NEW MICROALGAE), but due to the growth characteristics of the algae, a large culture area is still required to obtain solar energy. It is also reported that the synthesis of starch is studied in Saccharomyces cerevisiae (Recreating the synthesis of starch granules in yeast, 2015), *Escherichia coli,* etc., but the raw material used is agricultural starch or its hydrolyzed glucose, which does not conform to the objective of the present application.

Therefore, means of preparing starch at present still need to be improved.

### Summary

The present application aims to solve one of the technical problems in related technologies at least to some extent. Therefore, the present application provides a means for preparing starch by fixing carbon dioxide using non-optical energy.

In a first aspect of the present application, the present application provides a method for preparing starch using carbon dioxide. According to some embodiments of the present application, the method includes: (1) providing energy and carbon sources for microbial cells on the basis of carbon dioxide and extracellular non-optical energy; and (2) generating starch within the microbial cells on the basis of at least one of up-regulated glucose-1-phosphate adenylyltransferase and starch synthase in the microbial cells. In this way, by utilizing non-optical energy, such as electric energy or hydrogen energy, starch can be effectively prepared inside the microbial cells by fixing carbon dioxide, so that carbon dioxide is utilized scientifically to help carbon-neutral strategies and can be used for biological industrial synthesis of starch so as to realize "agricultural industrialization".

In a second aspect of the present application, the present application provides a recombinant microorganism for preparing starch using carbon dioxide. According to an embodiment of the present application, the recombinant microorganism has: (1) an enzyme system for obtaining energy and carbon sources on the basis of carbon dioxide and extracellular non-optical energy; and (2) at least one of up-regulated starch synthase and glucose-1-phosphate adenylyltransferase compared with a wild type of the microorganism. In this way, the recombinant microorganism can effectively implement the method for preparing starch provided in the first aspect. By utilizing non-optical energy, such as electric energy or hydrogen energy, starch can be effectively prepared inside the microbial cells by fixing carbon dioxide, so that carbon dioxide is utilized scientifically to help carbon-neutral strategies and can be used for biological industrial synthesis of starch so as to realize "agricultural industrialization".

It can be understood by those skilled in the art that the features and advantages described for the method for preparing starch in the first aspect are also applicable to the recombinant microorganism, and will not be repeated here.

In a third aspect of the present application, the present application provides a method for constructing the microorganism described in the second aspect. The method includes the following operation on a starting microorganism: up-regulating at least one of starch synthase and glucose-1-phosphate adenylyltransferase. In this way, the recombinant microorganism described in the second aspect can be effectively prepared, and then the recombinant microorganism can effectively implement the method for preparing starch provided in the first aspect. By utilizing non-optical energy, such as electric energy or hydrogen energy, starch can be effectively prepared inside the microbial cells by fixing carbon dioxide, so that carbon dioxide is utilized scientifically to help carbon-neutral strategies and can be used for biological industrial synthesis of starch so as to realize "agricultural industrialization".

It can be understood by those skilled in the art that the features and advantages described for the method and recombinant microorganism for preparing starch in other aspects are also applicable to the method for constructing the recombinant microorganism, and will not be repeated here.

Some of the additional aspects and advantages of the present application will be provided in the following description, and some will become apparent from the following description, or be learned by practice of the present application.

### Brief Description of Figures

The above and/or additional aspects and advantages of the present application will become apparent and easy to understand from the description of embodiments in conjunction with the following accompanying drawings.
FIG. 1 shows the destruction efficiency of GA gene with different SgRNAs according to one embodiment of the present invention;
FIG. 2 shows the destruction efficiency of GP gene with different SgRNAs according to one embodiment of the present invention;
FIG. 3 shows starch yields of GlgA and/or GlgC recombinant strains containing different promoter combinations according to one embodiment of the present invention;
FIG. 4 shows starch production effects after GA destruction on the basis of pHis-EcGIgA-EcGIgC recombinant strains according to one embodiment of the present invention; and
FIG. 5 shows starch production effects after GP destruction on the basis of pHis-EcGIgA-EcGIgC and GA destruction according to one embodiment of the present invention.

### Detailed Description

Embodiments of the present application will be described in detail below, and examples of the embodiments are shown in the accompanying drawings. The embodiments described below with reference to the accompanying drawings are examples, and are intended to explain the present application, but shall not be understood as a limitation on the present application.

### Definition

Unless otherwise specified, terms or expressions used herein have the following meanings.

The term "recombinant microorganism" used herein refers to a microorganism that is genetically modified or genetically transformed to change the sequence or expression of an endogenous nucleic acid, or to express an exogenous nucleic acid (the exogenous nucleic acid may be contained in a vector or integrated into a genome of the microorganism). The "change" indicates that the expression of genes, or the RNA molecular level, or the equivalent RNA molecular level encoding one or more polypeptides or polypeptide subunits, or the activity of one or more polypeptides or polypeptide subunits is up-regulated or down-regulated, so that the expression, level, or activity is greater or less than that observed in the absence of the change.

The term "starting strain (or starting microorganism)" used herein refers to a strain that can be genetically modified or genetically transformed to obtain a recombinant microorganism. For example, the starting strain or microorganism may be a wild-type strain, or a strain that has undergone one or more genetic modifications or genetic transformations in advance or whose nucleic acid has changed compared with the wild-type strain.

The term "exogenous" used herein refers to a substance that does not originally exist in the starting strain, or a substance that is different from the corresponding substance in the starting strain, such as a nucleic acid that does not exist in the starting strain, or a nucleic acid that has a mutation compared with the corresponding gene sequence in the starting strain. According to the embodiments of the present application, the "exogenous nucleic acid" includes a nucleic acid from the same species as the starting strain, and also includes a heterologous nucleic acid (i.e., a nucleic acid from a different species from the starting strain).

The term "nucleic acid" used herein can be used interchangeably with the term "polynucleotide" and refers to an organic polymer composed of two or more monomers (including nucleotides, nucleosides, or analogues thereof), including but not limited to a single-stranded or double-stranded, sense or antisense deoxyribonucleic acid (DNA) of any length, and a sense or antisense ribonucleic acid (RNA) including siRNA. The term "nucleotide" refers to any of a variety of compounds composed of ribose or deoxyribose bound to purine or pyrimidine bases and bound to phosphate groups, and the compounds are basic structural units of nucleic acids. The term "nucleoside" refers to a compound (such as guanosine or adenosine) composed of purine or pyrimidine bases combined with deoxyribose or ribose, and the compound exists in nucleic acids.

It should be understood that the meaning of "nucleic acid" described herein includes "gene", and the nucleic acid described herein further includes "vector" or "plasmid".

The term "gene" used herein refers to a polynucleotide encoding a specific amino acid sequence, and the polynucleotide contains all or some of one or more proteins or enzymes, and may include a regulatory (non-transcribed) DNA sequence, such as a promoter sequence, which determines, for example, under what condition the gene is expressed. Transcription regions of genes may include untranslated regions (including introns and untranslated regions (5'UTR and 3'UTR)) and encoding sequences.

The term "up-regulate" used herein indicates that the activity of a specific functional protein is increased compared with the corresponding activity of the starting strain, and its biological activity can be improved by increasing the expression quantity of the specific functional protein in microbial cells (for example, by additionally transferring an exogenous nucleic acid encoding the functional protein, or by adjusting regulatory elements for the functional protein to encode a nucleic acid, such as promoters), or by modifying the amino acid sequence of the functional protein.

The term "down-regulate" used herein indicates that the activity of a specific functional protein is decreased compared with the corresponding activity of the starting strain, and its biological activity can be reduced by reducing the expression quantity of the specific functional protein in microbial cells (for example, by knocking out a nucleic acid encoding the functional protein, or by adjusting regulatory elements for the functional protein to encode a nucleic acid, such as promoters), or by modifying the amino acid sequence of the functional protein.

The "up-regulate" or "down-regulate" mentioned herein may be constitutive, for example, due to stable permanent transgenic expression, or due to stable mutation of an encoded nucleic acid or a corresponding endogenous gene of a nucleic acid, or due to regulation on the expression or behavior of a gene conferring a polypeptide or used for polypeptide expression; or instantaneous, for example, due to instantaneous transformation or temporary addition of a regulator (such as an agonist or an antagonist); or inducible, for example, by carrying nucleic acid molecules under the control of inducible promoters or transforming inducible constructs and adding inducers.

The term "expression" used herein refers to transcription and/or translation of an encoding gene fragment or gene. Generally, the obtained product is mRNA or protein. However, the expression product may alternatively include functional RNA, such as antisense strand nucleic acid, tRNA, snRNA, rRNA, RNAi, siRNA, or ribozyme. The expression may be global, partial, or instantaneous.

Herein, for additional introduction of other exogenous nucleic acid, the exogenous nucleic acid can be integrated into a genome of a microorganism, so as to perform functions. For example, by inserting the exogenous nucleic acid (gene) into an integration vector, the integration vector is homologously recombined with the genome of microorganism, so as to integrate the exogenous nucleic acid (gene) into the genome of microorganism. In addition, the exogenous nucleic acid may alternatively be dissociated from the genome of the microorganism, for example, inserted into an expression vector, and can be expressed without integration into the genome. In order to conveniently maintain the expression vector such as plasmids not to be lost in the amplification process of the microorganism, a screening gene such as a drug resistance gene can be set on the expression vector, so that a corresponding drug such as penicillin or kanamycin can be added to a culture medium for the microorganism, and the corresponding expression vector can always be maintained not to be lost in the amplification process of the microorganism.

The term "block" used herein indicates that the original gene no longer performs its original function or its original function is down-regulated after being modified or transformed.

The term "promoter" used herein refers to a section of DNA sequences that an RNA polymerase recognizes, binds and initiates transcription. The promoter contains conserved sequences required by the RNA polymerase for specific binding and transcription initiation, and most of them are located upstream of transcription initiation points of structural genes. Usually, the promoter is not transcribed. The promoter is located in a regulatory sequences that controls gene expression, upstream of a gene transcription initiation site (toward a 5' direction of a DNA sense chain), and has a length of about 100-1000 base pairs. Promoters commonly used in microbial genetic engineering can be divided into three categories according to their modes of action and functions: constitutive promoters (which maintain continuous activity in most or all tissues), specific promoters (which are tissue-specific or specific in a development stage), and inducible promoters (which are regulated by external chemical or physical signals).

The term "operably connected with a promoter" used herein indicates that a nucleic acid molecule connected to a promoter can be controlled by the promoter.

The term "about" used herein indicates that an index value fluctuates within a range of 5%. For example, a value of about 100 indicates that the value may fluctuate up and down 100 by 5.

### Method for preparing starch using carbon dioxide

In a first aspect of the present application, the present application provides a method for preparing starch using carbon dioxide. According to some embodiments of the present application, the method includes: (1) providing energy and carbon sources for microbial cells on the basis of carbon dioxide and extracellular non-optical energy; and (2) generating starch within the microbial cells on the basis of at least one of up-regulated glucose-1-phosphate adenylyltransferase and starch synthase in the microbial cells. In this way, by utilizing non-optical energy, such as electric energy or hydrogen energy, starch can be effectively prepared inside the microbial cells by fixing carbon dioxide, so that carbon dioxide is utilized scientifically to help carbon-neutral strategies and can be used for biological industrial synthesis of starch so as to realize "agricultural industrialization".

According to the embodiments of the present application, the above method for preparing starch will be described in detail below.

It should be noted that "extracellular non-optical energy" used herein refers to energy that exists outside cells and can be utilized by microbial cells, such as thermal energy, electric energy, chemical energy (especially hydrogen energy), potential energy, and magnetic energy. According to some embodiments of the present invention, the non-optical energy that can be used includes, but is not limited to, at least one of hydrogen energy and electric energy. Therefore, the method can effectively break through the limitation of natural photosynthesis, create a more efficient artificial photosynthesis system, greatly improve the efficiency of biological carbon fixation, and realize rapid bio-transformation of carbon dioxide. According to the embodiments of the present invention, the ways of providing hydrogen energy and electric energy are not particularly limited, for example, the hydrogen energy is provided in a form of hydrogen and the electric energy is provided in a form of current.

It should be noted that the way of fixing carbon dioxide using extracellular non-optical energy such as hydrogen energy and electric energy is not particularly limited, and a microorganism can directly ingest hydrogen energy, for example, hydrogen is introduced into a culture environment for the microorganism, or a microorganism directly ingests electric energy, for example, current is charged into a culture environment for the microorganism. In addition, at least one of extracellular non-optical energy, such as hydrogen energy and electric energy, can chemically react with carbon dioxide outside cells, so that microorganism can further ingest a reaction product of the chemical reaction. According to some embodiments of the present application, step (1) may further include: enabling the microbial cells to ingest the carbon dioxide as a carbon source and absorb the extracellular non-optical energy; and/or obtaining a low-carbon compound on the basis of carbon dioxide using the reducing capability of the extracellular non-optical energy, and enabling the microorganism to ingest the low-carbon compound as a carbon source.

It should be noted that the term "carbon source" used herein refers to a material that contains a carbon element and can be used by a microorganism for growth and reproduction. It is well known to those skilled in the art that different microorganisms can use different carbon sources. The main functions of carbon sources on microbial growth and metabolism are to provide carbon skeletons for cells, provide energy required for cell life activities, and provide carbon skeletons for synthetic products. The carbon sources play an important role in preparing microbial culture media or cell culture media, and provide a material basis for the normal growth and division of microorganisms or cells. Therefore, when the "carbon source" is mentioned later, it may refer to carbon dioxide or low-carbon compounds synthesized by carbon dioxide with the help of electric energy and/or hydrogen energy. According to some embodiments of the present application, the low-carbon compound is at least one selected from carbon monoxide, formic acid, methanol, methane, ethanol, formaldehyde, acetaldehyde, acetic acid, propanol, propane, propionaldehyde, acetone, hydroxyacetone, dihydroxyacetone, and glycerol. Therefore, starch can be synthesized using microorganisms that can use these low-carbon compounds as carbon sources, especially the only carbon source. The efficiency of preparing starch can be further improved. It should be noted that the term "only carbon source" used here should be broadly understood, and some other carbon-containing materials can be added to activate relevant activities of microorganisms in the culture process, for example, appropriate carbon-containing components can be added to activate inducible promoters, for example, a small amount of methanol can be added to activate an AOX1 promoter. Of course, those skilled in the art can understand that in order to improve the growth rate of microorganisms, other conventional nutrients, such as nitrogen sources, can also be provided for the microorganisms. Details are not described herein.

In addition, according to some embodiments of the present application, the microorganism is at least one of modified yeast and bacteria to be suitable for using the carbon source for metabolism. For example, carbon dioxide can be fixed using non-optical energy such as electric energy or hydrogen energy by transforming *Escherichia coli* or yeast. For example, for microorganisms that cannot use electric energy, such as *Escherichia coli,* relevant enzyme systems of other microorganisms that can use electric energy can be transferred into *Escherichia coli* for expression, so that the *Escherichia coli* can use relevant carbon sources or energy. Therefore, according to the embodiments of the present application, *Escherichia coli* can be transformed into all biomass carbon generated from carbon dioxide, and industrial yeast Pichia pastoris can be transformed from heterotrophs into autotrophs that can grow by carbon dioxide, for example, *Escherichia coli* can be transformed into synthetic methylotrophic bacteria that can grow only by methanol. Specifically, an enzyme system related to Calvin carbon fixation cycle can be transferred into *Escherichia coli* or yeast species such as *Pichia pastoris,* or an enzyme system related to a XuMP pathway of *Pichia pastoris* can be transferred into *Escherichia coli.*

Those skilled in the art can synthesize low-carbon compounds, including but not limited to low-carbon compounds containing 1-3 carbon atoms, using hydrogen or current on the basis of carbon dioxide by any known method. For example, the low-carbon compound may be at least one selected from carbon monoxide, formic acid, methanol, methane, ethanol, formaldehyde, acetaldehyde, acetic acid, propanol, propane, propionaldehyde, acetone, hydroxyacetone, dihydroxyacetone, and glycerol. For example, hydrogen has a strong reducing capability, and can reduce carbon dioxide under appropriate reaction conditions to obtain carbon monoxide or hydrocarbons with controllable carbon hydrogen ratio. According to the embodiments of the present invention, hydrogen can be obtained by decomposing water with solar energy. In addition, as a non-limiting example, carbon dioxide can be hydrogenated with high selectivity and high stability to synthesize methanol through a bimetallic solid solution oxide (ZnO-ZrO₂) as a catalyst using carbon dioxide and hydrogen.

For the convenience of understanding, some types of microorganisms that can be used in the present application are listed below. According to some embodiments of the present application, the microorganism suitable for absorbing electric energy includes, but is not limited to, at least one of *Geobacter, Sporomusa, Methanogens, Acetobacterium,* etc.; the microorganism suitable for ingesting acetic acid as a carbon source includes, but is not limited to, *Yarrowia lipolytica;* the microorganism suitable for absorbing hydrogen energy includes, but is not limited to, at least one of hydroxide bacteria, *Ralstonia,* and *Clostridium;* the microorganism suitable for ingesting carbon monoxide as a carbon source includes, but is not limited to, *Clostridium;* the microorganism suitable for ingesting formic acid as a carbon source includes, but is not limited to, *Ralstonia;* the microorganism suitable for ingesting methanol as a carbon source includes, but is not limited to, at least one of *Pichia pastoris, Candida boidinii,* and *Hansenula polymorpha;* the microorganism suitable for ingesting methane as a carbon source includes, but is not limited to, *Methylobacter* or *Methylococcus;* or the microorganism suitable for ingesting ethanol as a carbon source includes, but is not limited to, *Saccharomyces cerevisiae.* Therefore, the efficiency of preparing starch can be further improved.

Further, after the carbon dioxide or the low-carbon compound obtained on the basis of carbon dioxide is ingested as a carbon source, the microorganism can synthesize substances necessary for microbial growth, such as saccharides, protein, fibers, glucose, and enzymes, through its intracellular metabolic pathways. The metabolic pathway mentioned here may be either possessed by the wild-type microorganism or expressed by introducing exogenous genes into microbial cells through genetic modification. According to some embodiments of the present invention, carbon dioxide can be transformed into intermediates for subsequent synthesis of starch, such as glucose-1-phosphate. For example, six carbon fixation pathways including a Calvin cycle, a 3-hydroxypropionic acid dual cycle, a Wood-Ljungdahl pathway, a reducing (reverse) TCA cycle, a dicarboxylic acid/4-hydroxybutyric acid cycle and a 3-hydroxypropionic acid/4-hydroxybutyric acid cycle and two artificial pathways including a CETCH pathway and a reductive glycine pathway have been found in nature to obtain dicarbon or tricarbon intermediates.

According to some embodiments of the present application, the microorganism uses the carbon dioxide or the low-carbon compound as a main carbon source.

According to some embodiments of the present application, after energy and carbon sources are provided for microbial cells on the basis of carbon dioxide and extracellular non-optical energy, up-regulated glucose-1-phosphate adenylyltransferase GIgC and starch synthase GIgA can be used to catalyze glucose-1-phosphate (G-1-P) in microbial cells, so as to obtain a final product starch. The preparation of starch using the same microbial cells improves the production efficiency and reduces the production cost.

Through research, the inventors of the present application found that in the absence of starch synthase and glucose-1-phosphate adenylyltransferase in wild-type microorganisms or starting strains, recombinant microorganisms can express related enzymes by introducing exogenous genes. When starch synthase and glucose-1-phosphate adenylyltransferase have been expressed in the starting strains or wild-type microorganisms, the expression efficiency can be further improved by additionally transferring encoding genes of exogenous starch synthase and glucose-1-phosphate adenylyltransferase or changing promoters or other regulatory elements. In other words, according to some embodiments of the present application, at least one of the starch synthase and the glucose-1-phosphate adenylyltransferase is up-regulated relative to a wild type of the microorganism. Therefore, relative to the starting strain, the preparation efficiency of starch can be further improved. According to some embodiments of the present application, the starch synthase and the glucose-1-phosphate adenylyltransferase are expressed by exogenous gene sequences, respectively. Therefore, relative to the starting strain, the preparation efficiency of starch can be further improved.

Accord to the embodiments of the present application, the adopted starch synthase has the follow amino acid sequences:







 (SEQ ID NO: 1)

According to the embodiments of the present application, a gene sequence of the starch synthase may be the following base sequences, and its expression efficiency in yeast, especially in *Pichia pastoris,* is obviously higher than that of other base sequences:

ATGCAAGTTTTGCATGTTTGTTCTGAAATGTTTCCATTGTTGAAAACGGGTGGTTTGGCGGACGT
CATTGGTGCTTTGCCTGCTGCTCAAATTGCTGATGGGGTGGATGCTAGAGTTTTGTTGCCTGCTTTTCCTG
ATATTAGAAGAGGTGTTACTGATGCTCAAGTTGTATCGAGAAGGGATACTTTTGCTGGTCATATTACTTT
GTTGTTTGGTCATTATAATGGTGTTGGTATTTATTTGATTGATGCTCCACATTTGTATGATAGACCTGGTT
CTCCATATCATGATACTAATTTGTTTGCTTATACTGATAATGTTTTGAGATTTGCTTTGCTCGGATGGGTT
GGTGCTGAAATGGCTTCTGGTTTGGATCCATTTTGGAGACCTGATGTTGTTCATGCTCATGATTGGCATG
CTGGTTTGGCTCCTGCTTATTTGGCTGCTAGAGGTAGACCTGCTAAATCTGTTTTTACTGTTCATAATTTG
GCTTATCAAGGTATGTTTTATGCTCATCACATGAACGATATCCAATTGCCATGGTCTTTCTTTAACATTCAC
GGTTTGGAATTTAACGGTCAGATTTCTTTTTTGAAAGCTGGTTTGTATTATGCTGATCATATTACTGCTGT
TTCTCCAACTTATGCTAGAGAAATTACTGAACCACAATTTGCTTATGGTATGGAAGGTTTGTTGCAACAA
AGACATAGAGAAGGTAGATTGTCTGGCGTTTTGAATGGTGTTGATGAGAAAATTTGGTCTCCTGAAACT
GATTTGTTGTTGGCTTCTAGATATACTAGAGATACTTTGGAAGATAAAGCTGAAAATAAAAGACAATTGC
AAATTGCTATGGGTTTGAAAGTTGATGATAAAGTTCCATTGTTTGCTGTCGTCTCTAGGTTAACTTCTCAA
AAAGGTTTGGATTTGGTTTTGGAAGCTTTGCCTGGTTTGTTGGAACAAGGTGGTCAATTGGCTTTGTTGG
GGGCTGGTGATCCTGTTTTGCAAGAAGGTTTTTTGGCTGCTGCTGCTGAATATCCTGGTCAAGTTGGTGT
TCAAATTGGTTATCATGAAGCTTTTTCTCATAGAATTATGGGTGGTGCAGACGTTATTTTGGTGCCATCTA
GATTTGAACCATGTGGTTTGACTCAATTGTATGGTTTGAAATATGGTACTTTGCCATTGGTTAGAAGAAC
TGGTGGTTTGGCTGACACTGTTTCTGATTGTTCTTTGGAAAATTTGGCTGATGGCGTTGCGTCTGGTTTT
GTTTTTGAAGATTCTAATGCTTGGTCTTTGTTGAGAGCTATTAGAAGAGCTTTTGTTTTGTGGTCTAGGCC

GTCTTTGTGGAGATTTGTTCAAAGACAAGCTATGGCTATGGATTTTTCTTGGCAAGTTGCTGCTAAATCTT
ATAGAGAATTGTATTATAGATTGAAATAA (SEQ ID NO: 2)

Accord to the embodiments of the present application, an amino acid sequences of the glucose-1-phosphate adenylyltransferase is as follows:
MVSLEKNDHLMLARQLPLKSVALILAGGRGTRLKDLTNKRAKPAVHFGGKFRIIDFALSNCINSGIRR MGVITQYQSHTLVQHIQRGWSFFNEEMNEFVDLLPAQQRMKGENWYRGTADAVTQNLDIIRRYKAEYVVI LAGDHIYKQDYSRMLIDHVEKGARCTVACMPVPIEEASAFGVMAVDENDKIIEFVEKPANPPSMPNDPSKSL ASMGIYVFDADYLYELLEEDDRDENSSHDFGKDLIPKITEAGLAYAHPFPLSCVQSDPDAEPYWRDVGTLEAY WKANLDLASVVPELDMYDRNWPIRTYNESLPPAKFVQDRSGSHGMTLNSLVSGGCVISGSVVVQSVLFSRV RVNSFCNIDSAVLLPEVWVGRSCRLRRCVIDRACVIPEGMVIGENAEEDARRFYRSEEGIVLVTREMLRKLGH KQER (SEQ ID NO: 3); gene sequences of the glucose-1-phosphate adenylyltransferase may be the following base sequences, and its expression efficiency in yeast, especially in *Pichia pastoris,* is obviously higher than that of other base sequences:

ATGGTTTCTTTGGAAAAAAATGATCATTTGATGTTGGCTAGACAATTGCCATTGAAATCTGTTGC

TTTGATCTTGGCTGGTGGTAGAGGTACTAGATTGAAAGATTTGACTAATAAAAGAGCTAAACCTGCTGT

ACATTTTGGTGGTAAATTTAGAATAATAGATTTTGCTCTGTCTAACTGCATTAATTCTGGTATTAGAAGAA

TGGGTGTTATTACTCAATATCAATCTCATACTTTGGTGCAACATATTCAAAGAGGATGGTCATTCTTTAAT

GAAGAAATGAATGAATTTGTTGATTTGTTGCCTGCTCAACAAAGAATGAAAGGTGAAAATTGGTATAGA

GGTACTGCTGATGCTGTTACTCAAAATTTGGATATTATTAGAAGATATAAAGCTGAGTATGTTGTTATAT

TGGCTGGGGATCATATTTACAAACAAGATTATTCTAGAATGTTGATTGATCATGTTGAAAAAGGTGCTAG

ATGTACTGTTGCTTGTATGCCTGTTCCAATTGAAGAAGCTTCTGCTTTTGGTGTTATGGCTGTTGATGAAA

ATGATAAAATTATTGAATTTGTTGAAAAACCTGCTAATCCACCATCTATGCCAAATGATCCATCTAAATCT

TTGGCTTCTATGGGTATTTATGTTTTTGATGCTGATTATTTGTATGAATTGTTGGAGGAGGATGATAGAG
ATGAAAACTCATCTCATGATTTTGGTAAAGATTTGATTCCAAAAATTACTGAAGCTGGTTTGGCTTATGCT
CATCCATTTCCATTGTCTTGTGTTCAATCTGATCCTGATGCTGAACCATATTGGAGAGATGTTGGTACTTT
GGAAGCTTATTGGAAAGCTAATTTGGATTTGGCTTCTGTTGATGAATTGGATATGTATGATAGAAAT
TGGCCAATTAGAACTTATAATGAATCTTTGCCACCTGCTAAATTTGTTCAAGATAGATCTGGTTCTCATGG
TATGACTTTAAATTCTTTGGTTTCTGATGGTTGTGTTATTTCTGGTTCTGTGGTTGTGCAAAGTGTTTTGTT
TTCTAGAGTTAGAGTTAATTCTTTTTGTAATATTGATTCTGCTGTTTTGTTGCCTGAAGTTTGGGTTGGTA
GATCTTGTAGATTGAGAAGATGTGTTATTGATAGAGCTTGTGTTATTCCTGAAGGTATGGTTATTGGTGA
AAATGCTGAAGAAGATGCTAGAAGATTTTATAGATCTGAAGAAGGTATTGTTTTGGTTACTAGAGAAAT
GTTGAGAAAATTGGGTCATAAACAAGAAAGATAA (SEQ ID NO: 4)

According to some embodiments of the present application, the starch synthase and the glucose-1-phosphate adenylyltransferase are independently operably connected with a constitutive promoter or an inducible promoter. Therefore, the preparation efficiency of starch can be further improved, and the controllability of starch preparation can be realized.

According to some embodiments of the present application, the inducible promoter is suitable for improving the transcription level under the induction of the carbon source. According to some embodiments of the present application, both the starch synthase and the glucose-1-phosphate adenylyltransferase are controlled by the inducible promoters. Therefore, the preparation efficiency of starch can be further improved.

According to some embodiments of the present application, the constitutive promoter includes at least one selected from the following: ZWF1, a glucose-6-phosphate 1-dehydrogenase promoter; TPI1, a triose phosphate isomerase promoter; GSH1, a glutathione synthase promoter; POR1, a mitochondrial porin promoter; TKL1, a transketolase promoter; PGD1, a 6-phosphogluconate dehydrogenase promoter; PGM1, a phosphoglycerate mutase promoter; PK, a pyruvate kinase promoter; and a GAP glyceraldehyde-3-phosphate dehydrogenase promoter.

According to some embodiments of the present application, the inducible promoter includes at least one selected from the following: CAT1: a catalase promoter; TAL1: a transaldolase 1 promoter; TAL2: a transaldolase 2 promoter; ALD 4: a mitochondrial aldehyde dehydrogenase 4 promoter; DAK1: a dihydroxyacetone kinase promoter; FDH1: a formate dehydrogenase promoter; ALD: a mitochondrial aldehyde dehydrogenase promoter; DAS1: a dihydroxyacetone synthase 1 promoter; DAS2: a dihydroxyacetone synthase 2 promoter; AOX1: alcohol oxidase 1 promoter; and AOX2: an alcohol oxidase 2 promoter. A methanol inducible promoter, such as AOX1, is preferred.

According to some embodiments of the present application, the metabolism of starch is blocked by at least one of glucan 1,4-α-glucosidase and glycogen phosphorylase. Specifically, the blocking on the metabolism of starch by at least one of glucan 1,4-α-glucosidase and glycogen phosphorylase is performed by mutating a gene encoding at least one of glucan 1,4-α-glucosidase and glycogen phosphorylase. Therefore, the accumulation of the generated starch can be further improved. According to the embodiments of the present application, a specific gene can be mutated by gene editing, such as inserting or deleting 1-3 bases. The term "gene editing" used herein indicates that a specific target gene (also called "target gene" or "target") of a genome of an organism can be modified (including but not limited to inserted, deleted, or substituted). According to the embodiments of the present invention, under the guidance of SgRNA (recognizing a target gene), a nuclease Cas9 as "molecular scissors" is used to produce a site-specific double strand break (DSB) at a specific position in the genome, which further induces the organism to repair DSB through non-homologous end joining (NHEJ) or homologous recombination (HR) (also called "HDR" (Homology directed repair)), so as to realize specific modification. In order to improve the efficiency of homologous recombination directed double-stranded DNA repair (HDR), an additional single-stranded oligodeoxyribonucleotide (ssODN) designed on the basis of a target gene sequence is added as a homologous recombination repair template.

According to some embodiments of the present application, the gene encoding the glucan 1,4-α-glucosidase is mutated using SgRNA selected from the following:
7: gagtcgataacgatctcctt;
10: gttgttgatgtagccgtcta; or
32: ggacgtgatcagggaacatg.
32: ggacgtgatcagggaacatg is preferred.

According to some embodiments of the present application, the gene encoding the glycogen phosphorylase is mutated using SgRNA selected from the following:
1-509: ggccacctccgactcaatca;
6-509: gttaataagagcgttgtcca; or
10-1018: gagaagtcaaactcggtggt.

Therefore, the efficiency of destructing the glucan 1,4-α-glucosidase and the glycogen phosphorylase can be improved, the accumulation of starch can be further improved, the preparation efficiency of starch can be further improved, and the preparation cost of starch can be reduced.

Those skilled in the art can understand that, after starch is synthesized in the microbial cells, the microbial cells can be lysed to collect the starch, or the microbial cells can be genetically modified to secrete the starch outside the cells, or the microbial cells can be directly used as an edible product. Preferably, according to some embodiments of the present application, the method further includes: collecting the starch after lysing the microbial cells. Therefore, the starch can be purified, which is beneficial to wide application of the starch obtained subsequently.

### Recombinant microorganism

In a second aspect of the present application, the present application provides a recombinant microorganism for preparing starch using carbon dioxide. According to an embodiment of the present application, the recombinant microorganism has (1) an enzyme system for obtaining energy and carbon sources on the basis of carbon dioxide and extracellular non-optical energy; and (2) at least one of up-regulated starch synthase and glucose-1-phosphate adenylyltransferase compared with a wild type of the microorganism. In this way, the recombinant microorganism can effectively implement the method for preparing starch provided in the first aspect. By utilizing non-optical energy, such as electric energy or hydrogen energy, starch can be effectively prepared inside the microbial cells by fixing carbon dioxide, so that carbon dioxide is utilized scientifically to help carbon-neutral strategies and can be used for biological industrial synthesis of starch so as to realize "agricultural industrialization".

According to an embodiment of the present application, the extracellular non-optical energy includes at least one of hydrogen energy and electric energy.

According to an embodiment of the present application, microbial cells are suitable for ingesting the carbon dioxide as a carbon source and absorbing the extracellular non-optical energy; and/or
the microorganism is suitable for ingesting a low-carbon compound as a carbon source, and the low-carbon compound can be obtained on the basis of carbon dioxide using the reducing capability of the extracellular non-optical energy.

According to an embodiment of the present application, the recombinant microorganism has at least one of down-regulated glucan 1,4-α-glucosidase and glycogen phosphorylase compared with the wild type of the microorganism.

According to an embodiment of the present application, the microorganism is at least one of modified yeast and bacteria to be suitable for using the carbon source.

According to an embodiment of the present application, the low-carbon compound contains 1-3 carbon atoms, and optionally, the low-carbon compound is at least one selected from carbon monoxide, formic acid, methanol, methane, ethanol, formaldehyde, acetaldehyde, acetic acid, propanol, propane, propionaldehyde, acetone, hydroxyacetone, dihydroxyacetone, and glycerol.

For the convenience of understanding, some types of microorganisms that can be used in the present application are listed below. According to some embodiments of the present application, the microorganism suitable for absorbing electric energy includes, but is not limited to, at least one of *Geobacter, Sporomusa, Methanogens, Acetobacterium,* etc.; the microorganism suitable for ingesting acetic acid as a carbon source includes, but is not limited to, *Yarrowia lipolytica;* the microorganism suitable for absorbing hydrogen energy includes, but is not limited to, at least one of hydroxide bacteria, *Ralstonia,* and *Clostridium;* the microorganism suitable for ingesting carbon monoxide as a carbon source includes, but is not limited to, *Clostridium;* the microorganism suitable for ingesting formic acid as a carbon source includes, but is not limited to, *Ralstonia;* the microorganism suitable for ingesting methanol as a carbon source includes, but is not limited to, at least one of *Pichia pastoris, Candida boidinii,* and *Hansenula polymorpha;* the microorganism suitable for ingesting methane as a carbon source includes, but is not limited to, *Methylobacter* or *Methylococcus;* or the microorganism suitable for ingesting ethanol as a carbon source includes, but is not limited to, *Saccharomyces cerevisiae.* Therefore, the efficiency of preparing starch can be further improved.

According to an embodiment of the present application, the microorganism is suitable for using the carbon dioxide or the low-carbon compound as a main carbon source.

According to an embodiment of the present application, the starch synthase and the glucose-1-phosphate adenylyltransferase are expressed by exogenous gene sequences, respectively.

According to an embodiment of the present application, the starch synthase and the glucose-1-phosphate adenylyltransferase are independently operably connected with a constitutive promoter or an inducible promoter.

According to an embodiment of the present application, the inducible promoter is suitable for improving the transcription level under the induction of the carbon source.

According to an embodiment of the present application, both the starch synthase and the glucose-1-phosphate adenylyltransferase are controlled by the inducible promoters.

According to some embodiments of the present application, the constitutive promoter includes at least one selected from the following: ZWF1, a glucose-6-phosphate 1-dehydrogenase promoter; TPI1, a triose phosphate isomerase promoter; GSH1, a glutathione synthase promoter; POR1, a mitochondrial porin promoter; TKL1, a transketolase promoter; PGD1, a 6-phosphogluconate dehydrogenase promoter; PGM1, a phosphoglycerate mutase promoter; PK, a pyruvate kinase promoter; and a GAP glyceraldehyde-3-phosphate dehydrogenase promoter.

According to some embodiments of the present application, the inducible promoter includes at least one selected from the following: CAT1: a catalase promoter; TAL1: a transaldolase 1 promoter; TAL2: a transaldolase 2 promoter; ALD 4: a mitochondrial aldehyde dehydrogenase 4 promoter; DAK1: a dihydroxyacetone kinase promoter; FDH1: a formate dehydrogenase promoter; ALD: a mitochondrial aldehyde dehydrogenase promoter; DAS1: a dihydroxyacetone synthase 1 promoter; DAS2: a dihydroxyacetone synthase 2 promoter; AOX1: alcohol oxidase 1 promoter; and AOX2: an alcohol oxidase 2 promoter. A methanol inducible promoter, such as AOX1, is preferred. According to an embodiment of the present application, a gene encoding at least one of the 1,4-α-glucosidase and the glycogen phosphorylase carries a mutant to block the metabolism of starch by at least one of the glucan 1,4-α-glucosidase and the glycogen phosphorylase.

According to some embodiments of the present application, the gene encoding the glucan 1,4-α-glucosidase is mutated using SgRNA selected from the following:
SgRNA 7: gagtcgataacgatctcctt (SEQ ID NO: 5),
SgRNA 10: gttgttgatgtagccgtcta (SEQ ID NO: 6), or
SgRNA 32: ggacgtgatcagggaacatg (SEQ ID NO: 7).
SgRNA 32: ggacgtgatcagggaacatg (SEQ ID NO: 7) is preferred.

According to some embodiments of the present application, the gene encoding the glycogen phosphorylase is mutated using SgRNA selected from the following:
SgRNA 1-509: ggccacctccgactcaatca (SEQ ID NO: 8),
SgRNA 6-509: gttaataagagcgttgtcca (SEQ ID NO: 9), or
SgRNA 10-1018: gagaagtcaaactcggtggt (SEQ ID NO: 10). Therefore, the efficiency of destructing the glucan 1,4-α-glucosidase and the glycogen phosphorylase can be improved, the accumulation of starch can be further improved, the preparation efficiency of starch can be further improved, and the preparation cost of starch can be reduced.

It can be understood by those skilled in the art that the features and advantages described for the method for preparing starch in the first aspect are also applicable to the recombinant microorganism, and will not be repeated here.

### Method for constructing the microorganism

In a third aspect of the present application, the present application provides a method for constructing the microorganism described in the second aspect, characterized by including the following operation on a starting microorganism: up-regulating at least one of starch synthase and glucose-1-phosphate adenylyltransferase. In this way, the recombinant microorganism described in the second aspect can be effectively prepared, and then the recombinant microorganism can effectively implement the method for preparing starch provided in the first aspect. By utilizing non-optical energy, such as electric energy or hydrogen energy, starch can be effectively prepared inside the microbial cells by fixing carbon dioxide, so that carbon dioxide is utilized scientifically to help carbon-neutral strategies and can be used for biological industrial synthesis of starch so as to realize "agricultural industrialization".

According to the embodiments of the present application, the above method for constructing the recombinant microorganism may further have the following additional technical features:
According to an embodiment of the present application, the method includes modifying a metabolic system of the starting microorganism, so as to have the enzyme system for obtaining energy and carbon sources on the basis of carbon dioxide and extracellular non-optical energy.

According to an embodiment of the present application, the extracellular non-optical energy includes at least one of hydrogen energy and electric energy.

According to an embodiment of the present application, the metabolic system of the starting microorganism is modified, so that
microbial cells are suitable for ingesting the carbon dioxide as a carbon source and absorbing the extracellular non-optical energy; and/or
the microorganism is suitable for ingesting a low-carbon compound as a carbon source, and the low-carbon compound can be obtained on the basis of carbon dioxide using the reducing capability of the extracellular non-optical energy.

According to an embodiment of the present application, the starting microorganism is at least one of yeast and bacteria to be suitable for using the carbon source.

According to an embodiment of the present application, the low-carbon compound contains 1-3 carbon atoms, and optionally, the low-carbon compound is at least one selected from carbon monoxide, formic acid, methanol, methane, ethanol, formaldehyde, acetaldehyde, acetic acid, propanol, propane, propionaldehyde, acetone, hydroxyacetone, dihydroxyacetone, and glycerol.

For the convenience of understanding, some types of microorganisms that can be used in the present application are listed below. According to some embodiments of the present application, the microorganism suitable for absorbing electric energy includes, but is not limited to, at least one of *Geobacter, Sporomusa, Methanogens, Acetobacterium,* etc.; the microorganism suitable for ingesting acetic acid as a carbon source includes, but is not limited to, *Yarrowia lipolytica;* the microorganism suitable for absorbing hydrogen energy includes, but is not limited to, at least one of hydroxide bacteria, *Ralstonia,* and *Clostridium;* the microorganism suitable for ingesting carbon monoxide as a carbon source includes, but is not limited to, *Clostridium;* the microorganism suitable for ingesting formic acid as a carbon source includes, but is not limited to, *Ralstonia;* the microorganism suitable for ingesting methanol as a carbon source includes, but is not limited to, at least one of *Pichia pastoris, Candida boidinii,* and *Hansenula polymorpha;* the microorganism suitable for ingesting methane as a carbon source includes, but is not limited to, *Methylobacter* or *Methylococcus;* or the microorganism suitable for ingesting ethanol as a carbon source includes, but is not limited to, *Saccharomyces cerevisiae.* Therefore, the efficiency of preparing starch can be further improved.

According to an embodiment of the present application, the microorganism is suitable for using the carbon dioxide or the low-carbon compound as a main carbon source.

According to the embodiment of the present application, the starch synthase and the glucose-1-phosphate adenylyltransferase are at least one of up-regulated starch synthases and glucose-1-phosphate adenylyltransferases expressed by exogenous gene sequences, respectively.

According to an embodiment of the present application, the starch synthase and the glucose-1-phosphate adenylyltransferase are independently operably connected with a constitutive promoter or an inducible promoter.

According to an embodiment of the present application, the inducible promoter is suitable for improving the transcription level under the induction of the carbon source.

According to an embodiment of the present application, both the starch synthase and the glucose-1-phosphate adenylyltransferase are controlled by the inducible promoters.

According to some embodiments of the present application, the constitutive promoter includes at least one selected from the following: ZWF1, a glucose-6-phosphate 1-dehydrogenase promoter; TPI1, a triose phosphate isomerase promoter; GSH1, a glutathione synthase promoter; POR1, a mitochondrial porin promoter; TKL1, a transketolase promoter; PGD1, a 6-phosphogluconate dehydrogenase promoter; PGM1, a phosphoglycerate mutase promoter; PK, a pyruvate kinase promoter; and GAP, a glyceraldehyde-3-phosphate dehydrogenase promoter.

According to some embodiments of the present application, the inducible promoter includes at least one selected from the following: CAT1: a catalase promoter; TAL1: a transaldolase 1 promoter; TAL2: a transaldolase 2 promoter; ALD 4: a mitochondrial aldehyde dehydrogenase 4 promoter; DAK1: a dihydroxyacetone kinase promoter; FDH1: a formate dehydrogenase promoter; ALD: a mitochondrial aldehyde dehydrogenase promoter; DAS1: a dihydroxyacetone synthase 1 promoter; DAS2: a dihydroxyacetone synthase 2 promoter; AOX1: alcohol oxidase 1 promoter; and AOX2: an alcohol oxidase 2 promoter. A methanol inducible promoter, such as AOX1, is preferred. According to an embodiment of the present application, a gene encoding at least one of the 1,4-α-glucosidase and the glycogen phosphorylase carries a mutant to block the metabolism of starch by at least one of the glucan 1,4-α-glucosidase and the glycogen phosphorylase.

According to some embodiments of the present application, the gene encoding the glucan 1,4-α-glucosidase is mutated using SgRNA selected from the following:
SgRNA 7: gagtcgataacgatctcctt (SEQ ID NO: 5),
SgRNA 10: gttgttgatgtagccgtcta (SEQ ID NO: 6), or
SgRNA 32: ggacgtgatcagggaacatg (SEQ ID NO: 7).
SgRNA 32: ggacgtgatcagggaacatg (SEQ ID NO: 7) is preferred.

According to some embodiments of the present application, the gene encoding the glycogen phosphorylase is mutated using SgRNA selected from the following:
SgRNA 1-509: ggccacctccgactcaatca (SEQ ID NO: 8),
SgRNA 6-509: gttaataagagcgttgtcca (SEQ ID NO: 9), or
SgRNA 10-1018: gagaagtcaaactcggtggt (SEQ ID NO: 10). Therefore, the efficiency of destructing the glucan 1,4-α-glucosidase and the glycogen phosphorylase can be improved, the accumulation of starch can be further improved, the preparation efficiency of starch can be further improved, and the preparation cost of starch can be reduced.

It can be understood by those skilled in the art that the features and advantages described for the method and recombinant microorganism for preparing starch in other aspects are also applicable to the method for constructing the recombinant microorganism, and will not be repeated here.

In addition, it can be understood by those skilled in the art that the present application further provides a kit for constructing the microorganism described in the second aspect. According to an embodiment of the present application, the kit includes an exogenous gene expression vector for enabling a starting microorganism to express at least one of starch synthase and glucose-1-phosphate adenylyltransferase; and optionally, a protein function blocking agent for blocking functions of at least one of glucan 1,4-alpha-glucosidase and glycogen phosphorylase. Therefore, by utilizing the kit, the aforementioned recombinant microorganism can be effectively constructed, and the recombinant microorganism can effectively implement the method for preparing starch provided in the first aspect. By utilizing non-optical energy, such as electric energy or hydrogen energy, starch can be effectively prepared inside the microbial cells by fixing carbon dioxide, so that carbon dioxide is utilized scientifically to help carbon-neutral strategies and can be used for biological industrial synthesis of starch so as to realize "agricultural industrialization".

According to an embodiment of the present application, the kit further includes a carbon source metabolic transformation vector for transforming a metabolic system of the starting microorganism, so as to have an enzyme system for obtaining energy and carbon sources on the basis of carbon dioxide and extracellular non-optical energy.

According to an embodiment of the present application, the starch synthase and the glucose-1-phosphate adenylyltransferase are independently operably connected with a constitutive promoter or an inducible promoter.

According to an embodiment of the present application, the inducible promoter is suitable for improving the transcription level under the induction of the carbon source.

According to an embodiment of the present application, both the starch synthase and the glucose-1-phosphate adenylyltransferase are controlled by the inducible promoters.

According to some embodiments of the present application, the constitutive promoter includes at least one selected from the following: ZWF1, a glucose-6-phosphate 1-dehydrogenase promoter; TPI1, a triose phosphate isomerase promoter; GSH1, a glutathione synthase promoter; POR1, a mitochondrial porin promoter; TKL1, a transketolase promoter; PGD1, a 6-phosphogluconate dehydrogenase promoter; PGM1, a phosphoglycerate mutase promoter; PK, a pyruvate kinase promoter; and a GAP glyceraldehyde-3-phosphate dehydrogenase promoter.

According to some embodiments of the present application, the inducible promoter includes at least one selected from the following: CAT1: a catalase promoter; TAL1: a transaldolase 1 promoter; TAL2: a transaldolase 2 promoter; ALD 4: a mitochondrial aldehyde dehydrogenase 4 promoter; DAK1: a dihydroxyacetone kinase promoter; FDH1: a formate dehydrogenase promoter; ALD: a mitochondrial aldehyde dehydrogenase promoter; DAS1: a dihydroxyacetone synthase 1 promoter; DAS2: a dihydroxyacetone synthase 2 promoter; AOX1: alcohol oxidase 1 promoter; and AOX2: an alcohol oxidase 2 promoter. A methanol inducible promoter, such as AOX1, is preferred. According to an embodiment of the present application, a gene encoding at least one of the 1,4-α-glucosidase and the glycogen phosphorylase carries a mutant to block the metabolism of starch by at least one of the glucan 1,4-α-glucosidase and the glycogen phosphorylase.

Therefore, the efficiency of destructing the glucan 1,4-α-glucosidase and the glycogen phosphorylase can be improved, the accumulation of starch can be further improved, the preparation efficiency of starch can be further improved, and the preparation cost of starch can be reduced.

According to an embodiment of the present application, the constitutive promoter includes at least one selected from GAP.

The inducible promoter includes at least one selected from methanol inducible promoters, preferably AOX1.

According to an embodiment of the present application, the protein function blocking agent is suitable for enabling at least one of the encoding 1,4-α-glucosidase and glycogen phosphorylase to carry a mutation to block the metabolism of starch by at least one of the glucan 1,4-α-glucosidase and the glycogen phosphorylase.

According to an embodiment of the present application, the protein function blocking agent includes the following SgRNA, thereby mutating a gene encoding the glucan 1,4-α-glucosidase using the SgRNA selected from the following:
SgRNA 7: gagtcgataacgatctcctt (SEQ ID NO: 5),
SgRNA 10: gttgttgatgtagccgtcta (SEQ ID NO: 6), or
SgRNA 32: ggacgtgatcagggaacatg (SEQ ID NO: 7).
SgRNA 32: ggacgtgatcagggaacatg (SEQ ID NO: 7) is preferred.

According to some embodiments of the present application, the protein function blocking agent includes the following SgRNA, thereby mutating a gene encoding the glycogen phosphorylase:
SgRNA 1-509: ggccacctccgactcaatca (SEQ ID NO: 8),
SgRNA 6-509: gttaataagagcgttgtcca (SEQ ID NO: 9), or
SgRNA 10-1018: gagaagtcaaactcggtggt (SEQ ID NO: 10).

It can be understood by those skilled in the art that the features and advantages described in other aspects for the method for preparing starch, the recombinant microorganism and the construction method therefor are also applicable to the kit for constructing the recombinant microorganism, and will not be repeated here.

Some of the additional aspects and advantages of the present application will be provided in the following description, and some will become apparent from the following description, or be learned by practice of the present application.

### Example 1: Preparation of starch using Pichia pastoris

### Synthesis of GlgA and GIgC genes

According to amino acid sequences information SEQ ID NO: 1 (AAC76454.1) of GlgA (starch synthase, EC 2.4.1.21) and amino acid sequences information SEQ ID NO: 3 (AAC76455.1) of GlgC (glucose-1-phosphate adenylyltransferase, EC 2.7.7.27) derived from *Escherichia coli* in GenBank, the inventors obtain two preferred gene sequences of SEQ ID NO: 2 and 4 by screening and optimizing for *Pichia pastoris.*

### Construction of GlgA and/or GlgC expression vectors

The following 5 expression vectors were constructed using a strong constitutive promoter pGap and a strong inducible promoter pAOX1, respectively. The 5 expression vectors were all constructed by using the same skeleton plasmid pHis.

| Plasmid | Promoter for EcGIgA | Promoter for EcGIgC | Abbreviation |
|---|---|---|---|
| pHis-EcGIgA | Inducible (AOX1) | - | A ^{inducible} |
| pHis-EcGIgC | - | Inducible (AOX1) | C ^{inducible} |
| pHis-EcGIgA-EcGIgC | Inducible (AOX1) | Inducible (AOX1) | A ^{inducible} + C ^{inducible} |
| pHis-EcGIgA-pGAP-EcGIgC | Inducible (AOX1) | Constitutive (GAP) | A ^{inducible} + C ^{constitutive} |
| pHis-pGAP-EcGIgA-EcGIgC | Constitutive (GAP) | Inducible (AOX1) | A ^{constitutive} + C ^{inducible} |

### Destruction of GA gene (CRISPR-Cas9)

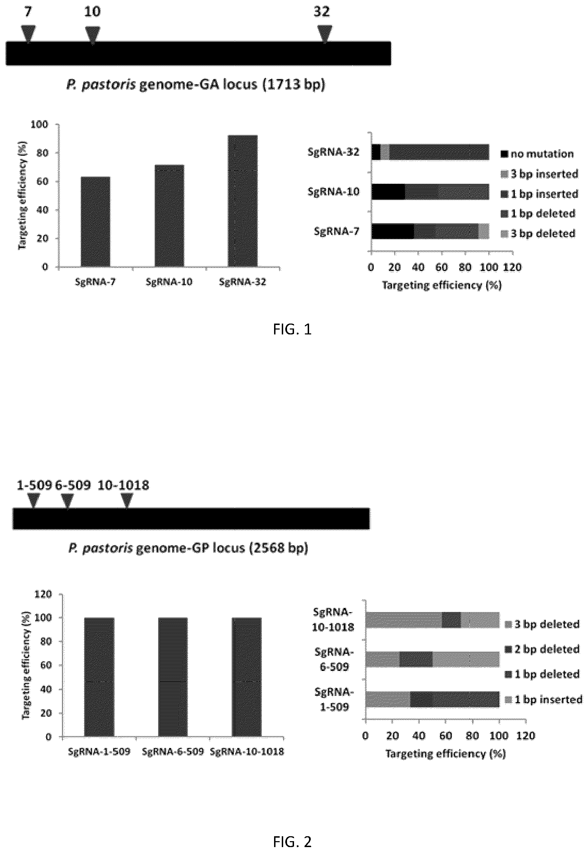

A GA (glucan 1,4-alpha-glucosidase, EC 3.2.1.3) gene on a genome of *Pichia pastoris* was destructed using CRISPR-Cas9 technology. According to a gene sequence of GA, 3 groups of different SgRNAs 7, 10, and 32 were screened out (their sequences were as mentioned above). As can be seen from FIG. 1, the destruction efficiency of GA varied among different SgRNAs, ranging from 64% to 92%, with 1 bp of insertion/deletion as main mutation.

### Destruction of GP gene (CRISPR-Cas9)

On the basis of destructing GA, a GP (glycogen phosphorylase, EC 2.4.1.1) gene on the genome of *Pichia pastoris* was further destructed using the CRISPR-Cas9 technology. According to a gene sequence of GP, 3 groups of different SgRNAs 1-509, 6-509, and 10-1018 were screened out (their sequences were as mentioned above). As can be seen from FIG. 2, 100% of destruction efficiency can be obtained for GP, without SgRNA difference but with balanced mutation diversity.

### Preparation of competent Pichia pastoris

*Pichia pastoris* strains GS115 were inoculated to 500 mL YPD medium, and cultured at 30°C and 200 r/min until OD600 = 1.3-1.5. Cells were collected by centrifugation at 4°C and 4500 r/min for 5 min, and washed once with 500 mL and 250 mL of pre-cooled sterile water and 20 mL of pre-cooled 1 mol/L sorbitol, respectively. After each washing, the cells were collected by centrifugation at 4°C and 4500 r/min for 5 min. Finally, the cells were suspended with 1 mL of pre-cooled 1 mol/L sorbitol, and dispensed into 80 µL/tube for fresh use, that is, electric shock-competent cells were obtained.

### Obtaining of recombinant Pichia pastoris strains

Five kinds of correctly constructed GlgA and/or GlgC expression vectors containing different promoter combinations, about 10 µg each, were linearized by *BspE* I digestion, and linear DNA was recovered by ethanol precipitation and dissolved in 10 µL sterile water. The linear DNA and 80 µL of GS115 competent cells were mixed and transferred to a pre-cooled 0.2 cm electric electroporation cup. Electric shock was performed using an electroporator of BIO-RAD Company in the United States according to the parameters of *Pichia pastoris* preset by the electroporator. After the electric shock, 1 mL of pre-cooled 1 mol/L sorbitol was immediately added to the electric shock cup, then all the solution in the electric shock cup was transferred to a sterile centrifuge tube, and 200-300 µL bacteria solution was directly taken to coat an MD plate (13.4 g/L yeast nitrogen base, 4×10⁻⁴ g/L D-biotin, 20 g/L glucose, and 15 g/L agarose). 1 µg DNA were used for the transformation of CRISPR-Cas9 related expression vectors, and the other steps were the same as above. After transformation, a YPD resistant plate containing G418 (500 µg/mL) was coated. Inverted culture was performed at 30°C for 2-4 days until colonies appeared, and recombinant strains can be obtained after PCR verification.

### Synthesis of methanol using carbon dioxide

Carbon dioxide was hydrogenated with high selectivity and high stability to synthesize methanol through a bimetallic solid solution oxide (ZnO-ZrO₂) as a catalyst using carbon dioxide and hydrogen. The produced methanol can be measured by gas chromatography (Agilent 7890B), where the chromatographic instrument was equipped with a thermal conductivity detector (TCD) and a flame ionization detector (FID), and the chromatographic column had a specification of 2 m × 3.175 mm (Agilent).

### Culture of recombinant Pichia pastoris strains

The recombinant strains were inoculated into a 25 mL BSM liquid medium (5 g/L ammonium sulfate, 3 g/L potassium dihydrogen phosphate, 0.5 g/L magnesium sulfate heptahydrate, 4 mL/L trace element mother solution (6 g/L copper sulfate pentahydrate, 0.08 g/L potassium iodide, 2.68 g/L manganese sulfate, 0.02 g/L boric acid, 0.2 g/L sodium molybdate dihydrate, 20 g/L zinc sulfate heptahydrate, 65 g/L ferrous sulfate heptahydrate, 0.916 g/L cobalt chloride hexahydrate, 5 mL/L sulfuric acid, and 0.2 g/L D-biotin), with a pH value adjusted to 6.0), and cultured by shaking at 30°C and 200 r/min for 48 h to obtain a seed solution. The seed solution was inoculated into fresh BSM, the initial OD600 was adjusted to 1, shaking culture was performed at 30 °C and 200 r/min, and methanol was added every day until the final concentration was 1% (V/V).

### Measurement of artificial starch yield

The concentration of cells cultured at 30°C for 2 d was adjusted to 10 OD/mL, and the cells were centrifuged and then re-suspended in a lysis solution (20 mM Tris-CI, 500 mM NaCl, pH 7.5) for ultrasonication. The ultrasonication procedure was as follows: ultrasonication for 5 s, pause for 5 s, and the total time was 10 min. 50 µL ultrasonicated solution was boiled at 100°C for 5 min and incubated with 30 U/mL α-amylase and 30 U/mL glucoamylase for a period of time until the starch was completely hydrolyzed into glucose, and then the content of glucose was tested by a glucose determination kit (APPLYGEN). The yield of starch was expressed by the content of glucose.

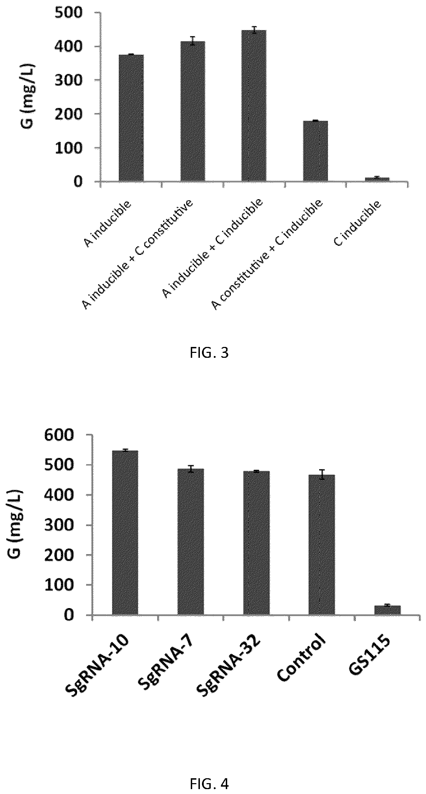

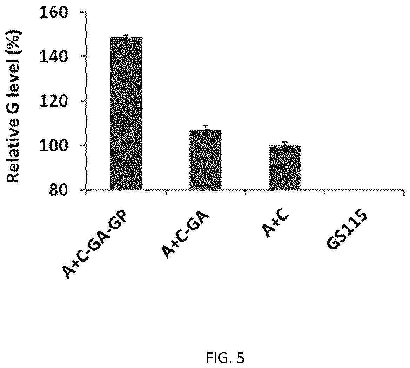

FIG. 3 shows starch yields of GlgA and/or GlgC recombinant strains containing different promoter combinations. FIG. 4 shows starch production effects after GA destruction on the basis of pHis-EcGIgA-EcGIgC recombinant strains. FIG. 5 shows starch production effects after GP destruction on the basis of pHis-EcGIgA-EcGIgC and GA destruction. The results showed that the highest yield of starch in artificial starch cell factories can reach 450 mg/L (60 mg/g DCW), with the highest yield of GS115/pHis-EcGIgA-EcGIgC (abbreviated as A+C). On this basis, a recombinant strain GS115/pHis-EcGIgA-EcGIgC-GP-1-509 (abbreviated as A+C-GP) was obtained by further knocking out a key enzyme gene GP for starch utilization. The recombinant strains A+C-GP and the starting strain A+C were simultaneously subjected to induced fermentation test, and compared with GS115, the results showed that the starch yield of A+C-GP was 150% higher than that of A+C and reached 750 mg/L (100 mg/g DCW).

### Example 2: Preparation of starch using hydrogen-oxidizing bacteria Cupriavidus necator

### Construction of recombinant hydrogen-oxidizing bacteria strains

According to sequences information (AAC76454.1) of GlgA (starch synthase, EC 2.4.1.21) and sequences information (AAC76455.1) of GlgC (glucose-1-phosphate adenylyltransferase, EC 2.7.7.27) derived from *Escherichia coli* in GenBank, two codon optimized gene sequences shown in SEQ ID NO: 11 and 12 were obtained for hydrogen-oxidizing bacteria *Cupriavidus necator,* GlgA and GIgC genes were cloned into pBBRI and pCM vectors respectively by simple cloning (You C, Zhang X-Z, Zhang Y-HP. 2012. Simple cloning via direct transformation of PCR product (DNA multimer) to Escherichia coli and Bacillus subtilis. Appl. Environ. Microbiol. 78(5):1593-1595.) to obtain corresponding recombinant plasmids pBBRI-GIgA and pCM-GIgC, then the recombinant plasmids were transformed into *Escherichia coli* S17, and finally the recombinant plasmids pBBRI-GIgA and pCM-GIgC were integrated into hydrogen-oxidizing bacteria *Cupriavidus necator.*

### Production of starch by recombinant strains using carbon dioxide

Hydrogen-oxidizing bacteria *Cupriavidus necator* can grow using carbon dioxide as the only carbon source. The recombinant strains were inoculated to 40 mL of medium (9.0 g/L sodium phosphate dibasic dodecahydrate, 1.5 g/L potassium dihydrogen phosphate, 1.0 g/L ammonium sulfate, 80 mg/L magnesium sulfate heptahydrate, 1 mg/L calcium sulfate dihydrate, 0.56 mg/L nickel sulfate heptahydrate, 0.4 mg/L ferric citrate, 200 mg/L sodium hydrogen carbonate, and 50 mg/L ferrous sulfate heptahydrate, with a pH value adjusted to 6.5) for culture, 1 mL/L of trace elements (100 mg/L zinc sulfate heptahydrate, 30 mg/L manganese chloride tetrahydrate, 300 mg/L boracic acid, 200 mg/L nickel chloride hexahydrate, and 30 mg/L sodium molybdate dihydrate) were added to the medium, and the initial OD was controlled to about 0.2, where the aeration ratio of H₂: O₂: CO₂ was 7: 1: 1, the aeration flow rate was about 20 mL/min, and the culture was performed at 30°C. Samples were collected every 24 h to measure OD and test starch yield.

The concentration of the cultured strains was adjusted to 10 OD/mL, and cells were centrifuged and then re-suspended in a lysis solution (20 mM Tris-CI, 500 mM NaCl, pH 7.5) for ultrasonication. The ultrasonication procedure was as follows: ultrasonication for 5 s, pause for 5 s, and the total time was 10 min. 50 µL ultrasonicated solution was boiled at 100°C for 5 min and incubated with 30 U/mL α-amylase and 30 U/mL glucoamylase for a period of time until the starch was completely hydrolyzed into glucose, and then the content of glucose was tested by a glucose determination kit (APPLYGEN). The yield of starch was expressed by the content of glucose.

The results showed that the strains constructed in this example can effectively produce starch using carbon dioxide.

### Example 3: Preparation of starch using Corynebacterium glutamicum

### Construction of Corynebacterium glutamicum chassis cells

According to sequences information (AAC76454.1) of GlgA (starch synthase, EC 2.4.1.21) and sequences information (AAC76455.1) of GlgC (glucose-1-phosphate adenylyltransferase, EC 2.7.7.27) derived from *Escherichia coli* in GenBank, two codon optimized genes were obtained for *Corynebacterium glutamicum,* and a recombinant expression vector pEC-XK99E-GIgA-GgIC was obtained with pEC as a skeleton vector, where GlgA and GlgC were expressed by the same promoter pTrc.

On this basis, the RpiB gene on the genome was destructed by homologous recombination to block the pentose phosphate pathway of the bacteria. Further, MDH, PHI and PHI genes required for the utilization pathway of ribulose monophosphate methanol were introduced to grow methanol-dependent strains.

### Preparation of competent Corynebacterium glutamicum

(1) Activation: *Corynebacterium glutamicum* (ATCC 13032) frozen at -80°C was streaked on an LB+0.5% glucose solid plate, and then cultured at 30°C to grow single colonies.
(2) Seed culture: A single colony was selected and inoculated into 10 mL of LB+0.5% glucose liquid medium, and cultured at 30°C and 200 r/min for about 12 h.
(3) Initial cells were transferred to 50 mL of liquid LB medium containing 3% of glycine and 0.1% of Tween 80, so that the OD600 of the initial cells reached 0.3.
(4) The cells were cultured at 30°C and 200 r/min until the OD600 reached 0.9. After cell culture, the bacteria solution was ice-bathed for 15 min, and then cells were collected at 4°C and 5000 r/min.
(5) The supernatant was discarded, and the cells were washed 4 times with 30 mL of pre-cooled 10% glycerol.
(6) The supernatant was discarded, and finally the cells were re-suspended with 0.2 mL of pre-cooled 10% glycerol, dispensed in 1.5 mL centrifugal tubes with 80 µL in each tube, and stored in a refrigerator at -80°C for later use.

### Obtaining of Corynebacterium glutamicum recombinant strains

Electroporation-competent cells frozen at -80°C were pipetted into each tube and melted on ice. 5 µL of correctly constructed recombinant expression vector was added, and the tube wall was flicked to mix the recombinant expression vector with the competent cells evenly. The mixture was transferred to a 1 mm pre-cooled electroporation cup, and electrically shocked at 1.8 kV. After the electric shock, 915 µL of BHI liquid medium (18.5 g/L brain heart infusion and 91 g/L sorbitol) was added immediately, the mixture was placed in a 46°C warm bath for 6 min and then transferred to 30°C resuscitation culture for 2 h, and finally an appropriate amount of bacteria solution was applied to an LBHIS (5 g/L peptone, 5 g/L sodium chloride, 2.5 g/L yeast extract, 18.5 g/L brain heart infusion, and 91 g/L sorbitol) solid plate containing 25 µg/mL kanamycin and inversely cultured at 30°C until colonies were grown. Corresponding recombinant strains can be obtained after PCR verification.

### Production of starch by Corynebacterium glutamicum using carbon dioxide

Carbon dioxide was hydrogenated with high selectivity and high stability to synthesize methanol through a bimetallic solid solution oxide (ZnO-ZrO₂) as a catalyst using carbon dioxide and hydrogen. The produced methanol can be measured by gas chromatography (Agilent 7890B), where the chromatographic instrument was equipped with a thermal conductivity detector (TCD) and a flame ionization detector (FID), and the chromatographic column had a specification of 2 m × 3.175 mm (Agilent).

The recombinant strains were inoculated into CGX11 (20 g/L ammonium sulfate, 5 g/L urea, 1 g/L potassium dihydrogen phosphate, 1.3 g/L dipotassium hydrogen phosphate trihydrate, 42 g/L MOPS, 0.01 g/L calcium chloride, 0.25 g/L magnesium sulfate heptahydrate, 0.01 g/L ferrous sulfate heptahydrate, 0.01 g/L manganese sulfate monohydrate, 0.001 g/L zinc sulfate heptahydrate, 0.2 mg/L copper sulfate, 0.02 mg/L nickel chloride hexahydrate, 0.2 mg/L D-biotin, 0.03 g/L protocatechuic acid, 0.1 mg/L vitamin B1, 5 g/L xylose, and 5 g/L methanol, with a pH value adjusted to 7.0), and cultured at 30°C and 200 r/min.

The concentration of the cultured strains was adjusted to 10 OD/mL, and cells were centrifuged and then re-suspended in a lysis solution (20 mM Tris-CI, 500 mM NaCl, pH 7.5) for ultrasonication. The ultrasonication procedure was as follows: ultrasonication for 5 s, pause for 5 s, and the total time was 10 min. 50 µL ultrasonicated solution was boiled at 100°C for 5 min and incubated with 30 U/mL α-amylase and 30 U/mL glucoamylase for a period of time until the starch was completely hydrolyzed into glucose, and then the content of glucose was tested by a glucose determination kit (APPLYGEN). The yield of starch was expressed by the content of glucose. The results were shown in Table 1.

**Table 1. Levels of starch accumulation by fermentation of recombinant Corynebacterium glutamicum strains heterogeneously expressing glucose-1-phosphate adenylyltransferase (EC 2.7.7.27) and starch synthase (EC 2.4.1.21)**

| Methanol: xylose (molar ratio) | Starch accumulation (shown in glucose equivalent) |
|---|---|
| 0: 1 | - |
| 1: 1 | + + |
| 3.83: 1 | + + + |
| 1: 0 | - |

| | |
|---|---|
| Note: More + indicates more starch accumulation. | |

The results showed that *Corynebacterium glutamicum* constructed in this example can effectively produce starch using methanol.

### Example 4: Preparation of starch using Escherichia coli

### Construction of recombinant Escherichia coli strains

According to sequences information (AAC76454.1) of GlgA (starch synthase, EC 2.4.1.21) and sequences information (AAC76455.1) of GlgC (glucose-1-phosphate adenylyltransferase, EC 2.7.7.27) derived from *Escherichia coli* in GenBank, primers were designed respectively, and GIgA and GIgC genes were cloned into a pET21b vector respectively by restriction enzyme ligation to obtain corresponding recombinant plasmids pET21b-GlgA and pET21b-GlgC. The two plasmids were transformed into *Escherichia coli* BL21(DE3) by chemical transformation to obtain corresponding recombinant strains.

### Production of starch by recombinant Escherichia coli strains using different carbon sources

Single colonies were selected, cultured in LB (0.5% yeast extract, 1% sodium chloride, and 1% tryptone) at 37 °C and 220 r/min overnight, and then transferred in an inoculation quantity of 1% to a medium containing different carbon sources (M9 as the basic medium; glucose, glycerol, and DHA as the carbon sources; and LB as the control) for culture until OD600=0.8, and 0.5 mM IPTG was added for induced fermentation at 16°C.

The concentration of the cultured strains was adjusted to 10 OD/mL, and cells were centrifuged and then re-suspended in a lysis solution (20 mM Tris-CI, 500 mM NaCl, pH 7.5) for ultrasonication. The ultrasonication procedure was as follows: ultrasonication for 5 s, pause for 5 s, and the total time was 10 min. 50 µl ultrasonicated solution was boiled at 100°C for 5 min and incubated with 30 U/mL α-amylase and 30 U/mL glucoamylase for a period of time until the starch was completely hydrolyzed into glucose, and then the content of glucose was tested by a glucose determination kit (APPLYGEN). The yield of starch was expressed by the content of glucose. The results were shown in Table 2.

**Table 2. Levels of starch accumulation by fermentation of recombinant Escherichia coli strains**

| Carbon source | Glucose-1-phosphate adenylyltransferase (GIgC) | Starch synthase (GIgA) | Starch accumulation (mg/L) |
|---|---|---|---|
| LB | - | + | 115 |
| LB | + | - | 86 |
| Glycerin | - | + | 896 |
| Glycerin | + | - | 247 |
| DHA | - | + | 12.6 |
| Glucose | - | + | 240 |
| Glucose | + | - | 35 |
| CK | - | - | 5.6 |

The results showed that the *Escherichia coli* constructed in this example can effectively produce starch using different carbon sources.

### Example 5: Preparation of starch using Yarrowia lipolytica

### Construction of recombinant Yarrowia lipolytica strains

According to 9 pairs of amino acid sequences (Table 3) of exogenous glucose-1-phosphate adenylyltransferase (EC 2.7.7.27) and starch synthase (EC 2.4.1.21), codons of gene sequences were optimized by *Yarrowia lipolytica* as a host, and gene fragments were synthesized into integrated expression plasmids. The two genes were respectively located between a promoter PrTef and a terminator Tlip2 and between a promoter PrGPD and a terminator Tcyc, to form 9 double expression cassette plasmids. The plasmids were digested with double enzymes to obtain an integrated DNA fragment. The integrated DNA fragment was integrated into a *Yarrowia lipolytica* chromosome by chemical transformation to obtain recombinant *Yarrowia lipolytica* strains.

**Table 3. Amino acid sequences (SEQ ID NO: 13-30 sequentially) of glucose-1-phosphate adenylyltransferase (EC 2.7.7.27) and starch synthase (EC 2.4.1.21) used in the present invention**

| Name of protein | | Amino acid sequences |
|---|---|---|
| Gl uc os e-1-ph os ph ate ad en yly ltr an sfe ras e (EC 2.7 .7. 27) | CsGI gC1 | MKAQPPLRLTAQAMAFVLAGGRGSRLKELTDRRAKPAVYFGGKARIIDFALSNAMNSGIRKMAI ATQYKAHSLIRHIQRGWNFFREERNEYLDILPASQRVDENRWYLGTADAVTQNIDIVDSYDIKYV IILAGDHVYKMDYEIMLRQHCETGADVTIGCLTVPRAEATAFGVMHVDANLRITDFLEKPADPP GIPGDEANALASMGIYVFDWAFLRDLLIRDAEDPNSSHDFGHDLIPAIVKNGKAMAHRFSDSCV MTGLETEPYWRDVGTIDAFWQANIDLTDFTPKLDLYDREWPIWTYSQIVPPAKFIHDSENRRGT AISSLVSGDCIVSGSEIRSSLLFTGCRTHSYSSMSHVVALPHVTVNRKADLTNCVLDRGVVVPEGL VIGQDAEEDARWFRRSEGGIVLVTQDMLDARARALN |
| | EcGI gC | MVSLEKNDHLMLARQLPLKSVALILAGGRGTRLKDLTNKRAKPAVHFGGKFRIIDFALSNCINSGI RRMGVITQYQSHTLVQHIQRGWSFFNEEMNEFVDLLPAQQRMKGENWYRGTADAVTQNLDI IRRYKAEYVVILAGDHIYKQDYSRMLIDHVEKGARCTVACMPVPIEEASAFGVMAVDENDKIIEF VEKPANPPSMPNDPSKSLASMGIYVFDADYLYELLEEDDRDENSSHDFGKDLIPKITEAGLAYAH PFPLSCVQSDPDAEPYWRDVGTLEAYWKANLDLASVVPELDMYDRNWPIRTYNESLPPAKFVQ DRSGSHGMTLNSLVSGGCVISGSVVVQSVLFSRVRVNSFCNIDSAVLLPEVWVGRSCRLRRCVID RACVIPEGMVIGENAEEDARRFYRSEEGIVLVTREMLRKLGHKQER |
| | SeGI gC | MVSLEKNDRLMLARQLPLKSVALILAGGRGTRLKDLTNKRAKPAVHFGGKFRIIDFALSNCLNSGI RRIGVITQYQSHTLVQHIQRGWSLFSEEMNEFVDLLPAQQRMQGENWYRGTADAVTQNLDIIR RYKAEYVVILAGDHIYKQDYSRMLIDHVEKGARCTVACMPVPIKEATAFGVMAVDESEKIIDFVE KPANPPAMPGDDSKALASMGIYVFDADYLYELLAADDKDDASSHDFGKDIIPKITREGMAYAHP FPLSCVQSDPDAEPYWRDVGTLEAYWKANLDLASVTPELDMYDQNWPIRTHMESLPPAKFVQ DRSGSHGMTLNSLVSGGCIISGSVVVQSVLFPRVRINSFCNIDSAVLLPEVWVGRSCRLRRCIIDR ACIIPEGMVIGENAEEDARRFYRSEEGIVLVTREMLRKLQVKQER |
| | YpGI gC | MVRFESTDSLMLARQLPNKTVALILAGGRGSRLKDLTATRAKPAVHFGGKFRIIDFALSNCLNSG VRRIGVITQYQSHTLVQHIQRGWSFLNEEMNEFVDLLPAQQRLSTEQWYKGTADAVCQNLDIIR RYDAEYIVILAGDHIYKMDYSRMLLDHVEKGAECTVACIPVPISEGSEFGIMEVTADYQITAFYEKP ANPPPIPGDPSNALASMGIYIFNADYLFKLLEEDNNTPGSSHDFGKDIIPQLTARKVVWAHPFDLS CVTSNAELPPYWRDVGTLDAYWRANLDLASVTPELDMYDRAWPIRTHMEPLPPAKFVQDRSG SHGMTMNSLVSGGCIVSGSVVVHSVLFPRVRVNSFCTIDSSLLLPDVHVGRSCRLRRCIIDRACHI PEGMVIGENADEDNARFYRSEGGGGVSDSGYAGKVRGKIEPLGFLFVRLDLLIRLSLLIRLNLFIR |
| | PIGI gC | MNLLIILTLFFKLASIQASH MRKVLALILAGGHGKRLGVLTEKIAKPAVPFGGKYRLIDFTLSNCVNSGIYHVGVLTQYRPHLLNN HINIGRPWDLDRKKGGVTILPPYLGGVAGWYRGTANAVYQNIEYVDSADPDFVLILSGDHVYA MDYNDIIDFHILKGAEGTIACIEVPPEETNRFGIMMTDLDSRIVDFEEKPQRARSNLASLGIYVFN WKFLKEYLIRDEQNNESTHDFGHDIIPLMINEGCQIYAFKFNGYWRDVGTVRSYWESNLELTRPI PPLNLYDRHWRFFTQTEEMPPAYCAPTSKIVNSIISEGCEIYGFVENSVIFQGVYIGENAVVKNSV VMTSTKIGENCVITDAVIAERVIVENRVIIGEGDDAKNKLDSDVYTGQITVIGMYSVIPSGSVIGKN CVVGVGVEKGDFKTQKVESGDYILHKE |
| | BcGI gC | MAQKQKCVAMLLAGGKGSRLSALTKNLAKPAVPFGGKYRIIDFTLSNCANSGIETVGILTQYQPL ELHNYIGIGNAWDLDRVSGGVTVLPPYAESSGVKWYTGTASAIYQNLNYLSQYEPEYVLILSGDHI YKMDYSKMLDYHIEKEADVSISVIEVPWDEASRFGIMNTNEEMEIVEFEEKPQFPRSNLASMGIY IFNWAILKEYLEMDARNPESSNDFGKDVLPLLLDEGKKLMAYPFEGYWKDVGTVKSLWEANMD |
| | MtG IgC | LLRDETSLNLNDRDWRIYSVNPNEPPQYIAEKARVEESLINEGCVIEGDVKHSVLFQGVTVEEGS MVIDSVVMPGAKIGKNVVIERAIVGSEMVIEDGTIIRPEKNVDDVVLIAEGK MREVPHVLGIVLAGGEGKRLYPLTADRAKPAVPFGGAYRLIDFVLSNLVNARYLRICVLTQYKSHS LDRHISQNWRLSGLAGEYITPVPAQQRLGPRWYTGSADAIYQSLNLIYDEDPDYIVVFGADHVYR MDPEQMVRFHIDSGAGATVAGIRVPRENATAFGCIDADDSGRIRSFVEKPLEPPGTPDDPDTTF VSMGNYIFTTKVLIDAIRADADDDHSDHDMGGDIVPRLVADGMAAVYDFSDNEVPGATDRDR AYWRDVGTLDAFYDAHMDLVSVHPVFNLYNKRWPIRGESENLAPAKFVNGGSAQESVVGAGS IISAASVRNSVLSSNVVVDDGAIVEGSVIMPGTRVGRGAVVRHAILDKNVVVGPGEMVGVDLEK DRERFAISAGGVVAVGKGVWI |
| | SoGI gC | MSNVRYISNLTRETYALILAGGRGSRLHELTDWRAKPALYFGGKFRIIDFPLSNCINSGIRRVGVVT QYKSHSLIRHVMRGWGHFKKELGESVEILPASQRYSENWYQGTADAVFQNIDIIRHELPKYVMV LSGDHVYRMDYAGLLAAHAESGADMTVSCLEVPVAEAAGAFGVMEVDDDMRILGFEEKPQLP KHCPGNPEKCLASMGNYVFNTEFLFEQLKKDAQNAESDRDFGKDIIPSIIEKHKVFAYPFKSAFPN EQAYWRDVGTLDSFWLANMELLSPTPALNLYDAKWPIWTYQEQLPPAKFVFDDDDRRGMAV DSIISGGCIISGATVRRSVLFNEVRVCSYSVVEDSVVLPDVVVLRHCKIKNAIIDRGCIIPEGTVIGYN HDHDRAKGFRVSEKGITLVTRDMLGLPVGYE |
| | SsGI gC | MVSLEKNDHLMLARQLPLKSVALILAGGRGTRLKDLTNKRAKPAVHFGGKFRIIDFALSNCINSGI RRMGVITQYQSHTLVQHIQRGWSFFNEEMNEFVDLLPAQQRMKGENWYRGTADAVTQNLDI IRRYKAEYVVILAGDHIYKQDYSRMLIDHVEKGARCTVACMPVPIEEASAFGVMAVDENDKIIEF VEKPANPPSMPNDPSKSLASMGIYVFDADYLYELLEEDDRDENSSHDFGKDLIPKITEAGLAYAH PFPLSCVQSDPDAEPYWRDVGTLEAYWKANLDLASVVPELDMYDRNWPIRTYNESLPPAKFVQ DRSGSHGMTLNSLVSGGCVISGSVVVQSVLFSRVRVNSFCNIDSAVLLPEVWVGRSCRLRRCVID RACVIPEGMVIGENAEEDARRFYRSEEGIVLVTREMLRKLGHKQER |
| Sta rch sy nt ha se (EC 2.4 .1. 21) | CsGI gA | METARGRVLSVASECVPLLKTGGLADVVGALPGALAAEGWEMRVLMPCYRPLKWRLEEMEEV FAEEDLFGGPGRVMAGEVAGRKMLLLDAPHLYDREGGPYAGPDGDWGDNAQRFAALSWIGA RIAREGLGDGWRPDVVHAHDWQAGFTPAYMNYWGSGGASSVLTVHNIAFQGWAPASLLSAL RLPPQEFHPAALEYYGGLSSLKAGLVSADHITTVSPTYACELMRPEFGMGLQGVIAQRAGQVTGI LNGVDTDIWSPEVEERPYDAESLKAKAENRAVLSGAFKLSVPGPLAILVSRLTYQKGIDLIPEVLPD FIAAGGGLAVLGTGDAPLEAAMRELEVRFPGRVGVRIGYDEGLSHLMFAGGDAVLVPSRFEPCG LTQMYGLRYGAIPVVALTGGLADTIINANPAAMAAGCATGLTFHPTEPPAFAEALRRLIHLYADP AAWETVQRNAMRHPVGWETSAAAYAALYRELVA |
| | EcGI gA | |
| | DdG IgA1 | MIFGTPNSTFLENRLGSSTGMPFSSATSLNSGVDQMMNELNIQQPTSHTVLFDLSWEVAKKVG GIYTVLKTKAPVTVEEYKSRYALIGPYNASTAPTEFEPLIPGPLSSPIIENMMKKYGIHVHFGKWLV EGYPKVFLIDLHSSMHKLGEWRWDLMSGFEQAGDNETNETIVFGYQSALLLKEFAEANPNDKYI AHFHEWQASVGLILLKKWKVPVSTIFTTHATLLGRYLAAGGVDLYNQMQVLNMDFEASKRGIY |
| | | HRHWIEKKSAADSHVFTTVSEITGYESEHILMRKPDVILPNGLKLDKFTALHEFQNLHAKYKGVLN EFVRGHFYGHYSDFDLDNTLYVFTAGRHEYFNKGVDMFLDSLAGLNKLLQQSGSKMTVVAFIIM PAATNNFNVESLKGHSYLKDMRRTCNTIVEAMGERLFEATSRGKMISPEELLSQEDLVMLKRRIF ALKQKSSGPPVVTHNMINDNDEILQHIRRIKLFNSQEDRVKVIYHPEFLTSTNPLIPLDYTEFVRGC HLGIFPSYYEPFGMTPAECCASGCPSITSNLTGFANYMSRALQDTDSKGIFIVDRRFKSSRETVDQ MTQYLWKFTQLDRRQRIELRNATEKLSELLDWRTLGKFYKTARALALERAFPPKPISRSPSPSPSSS LKLSTGLSNQIELQQQQQQQQPQPIGTTINLIPPSSNVSVTPTTTPTTTTTATTATTAPITTPKPNV IPINTGKENITLLSPNSMSSLLSDSLNEFKKQQQQQQQSKTPTTPTTTSTTTTTPSTTAAATNKSVL SNPTPTPSPNTSSFIPTNKGSTATTTTTTATPTPTPSNNTNGKPFNPIEALTKSNSSSNFATTSTAS VNTNNGGTNSPVSKSIPIPSSKSLK |
| | KoGI gA | MKILFVSYEVYPLAKVGGLADVAGSLPKYLEKEGVSVQIAMPFHKKVKADNIENTGIVITTKHLPE KYSFEIYKTPLPGSGVPVFLFKNDQLIDSDEVYEGTDLALQAIAFSDAVVKFAETLEPDLLHVNDW QPALIPAYISAFYNGKPKTLLTIHNLGYQGEFDKSYFYKTGLPEKLWQEGKAVKNGAFNFLKTGIV TATAISTVSPTYAKEIQTPEYGAGLDETLRALSDRLFGILNGIDYSEYNPATDKRIPVNFDINSLEKKK ENKIALQKELGLPVIDVPVIGLISRLVEQKGFDLIEAAAEKILSNDLQFVVLGTGEERYERLFKSLGER FPEKVSANITFNVDLAQKIYAGSDMFLMPSRYEPCGLGQMFAMRYGTIPVVRFTGGLRDTVKEF NPETLEGNGFGFEEYNPDKLLEAVEKAIKIYENKTLWNQLMLNAMNTDCSWDKSAREYIKLYKH VLNSGRDILNA |
| | SmG IgA | MKILFVAAEGAPFAKTGGLGDVIGALPKSLVKNNNEVSVILPYYDVVDAKFGDQIEDLFYFFTNV GWRREYVGIKHIFRDGVDFYFIDNKHYFYRGQIYGEFDDGERFAYFQLAALEAMEKIQFIPDILHV HDYHTAMIPYLLKEKYHWINAYHGIKTVFTIHNIEFQGQFNPSMLGELFGVGDERYRDGTLRWN DCLNWMKAAVLYADRVTTVSPSYAKEIMTPEFGKGLDQIMRMESGKLSGVVNGIDTDLFDPET DPHLAVHFSKDDLSGKAKNKAALQERVGLPVREDVPLVGIVSRLTDQKGFQLVVDQLNTMMQL DLQIVLLGTGYADFENAFAWFGHAYPDKMSANITFDLELAQQIYAASDIFLMPSAFEPCGLSQM MAMRYGTLPLVHEVGGLRDTVIPYNEFEKTGTGFGFQDFSGYWLTKTLEAALDVYYNRKEDWK ILQKNAMTTDFSWDTASQSYEHLYKELA |
| | YpGI gA | MRVLHVCSELFPLLKTGGLADVIGALPAAQLAEGADVRIILPAFPDLRRGIPETVLVREIDTFAGRV ALRYGHYRGIGIYLIDAPALYDRAGSPYHDASLYAYSDNYLRFALLGWMACELACGLDGYWRPE VVHAHDWHAGLTCAYLAARGRPARSVFTVHNLAYQGLFSADHLSELHLPAEFFQIYGLEFYGQIS YLKAGLFFADHVTTVSPTYAKEITQPAFGYGMEGLLQALARQGRLTGILNGVDSDIWDPQSDTLL PTRYDAENLQAKAINKTHLQTAMGLQLAENKPIFAVVSRLTVQKGLDLVLEALPELLALGGQLVV LGSGDATLQEAFLAAAAEHSGQVGVQIGYHEAFSHRIIAGSDVILVPSRFEPCGLTQLYGLKYGTL PLVRHTGGLADTVVDCALENLADGSASGFVFNECEAQALVKAIRRAFVLWSRPKHWRHVQRH AMRLDFGWQLAAVDYLSLYRRL |
| | DdG IgA2 | MRVLHVTSEVFPFSRSGGLGDVLGALPAVQATLGAQVSVLSPWYGSLAGAPEQIWAGDVADV GPVRVGELRQDGVRFLFLGLPEFEREGLYHPDDVWRFCTYGRAVLPVLRALNEVPDVLHGHDW QAGLVVAHAHEAGWRTVYTVHNLQYQGRWNLAEASGWSGLGPAWLTHEGAEFYGDLNLMK AGLIAANHVTTVSPQYAREITTQQYGEGLQGVLLRLTLEGRLSGIINGLDQERWNPRTDPDVPAY SDLAGKAAATQALRTEFGLDKAPVLGVVSRLADQKGMDLLIEALPRLVHNWNVVVLGGGDPLL TAALEGWAQHPRVSFAQGMNEALAHQIYAGSDAFAMPSRFEPCGLSQMIAMRYGTLPVVRET GGLVDTVPPDVGFRFQPATPEALVEACQQARAAFEDQSDWEARVARAMALDFSWDGPAREY LALYERVVTG |
| | PpGI gA | MATKKTSLTKHPLKILFVSSEVEGFAKTGGLADVAKSLPAALKKMGHDVRIVMPFYQTINGKDN AVAILSTELLVESQPFAVSYQVMQLDEGNVPVYALDAPQYYDRPELYAENNQAYADNGERFTFL |
| | | SAASLDLCEKLGFQPDVIHCNDWHTGLIPFLLKTRYAESDFFAKSKSVITIHNAVFKGVFNYDQYSL IPELIQRRYINAEMDPSHISMLKAGVAYADKVNAVSPNYASELLTHLGSHGMEADFQNRAKDLY GIINGCDYDDWNPETDVYIKQKFKANKVSLARGKKACRRDLQKQVNLPEVDVPVYGMVCRLTE QKGLHYLIPVLEDFLLNDVQVVIVGTGDPTLASALRDISEQHSDKFAFVETYSNPLAHCVEAGVDF FMMPSEFEPCGLNQMYSLAYGTLPIVRSVGGLKDTVIDYDQTPQVATGFIYDTPTPEALLIKLQRS LLLYCQRPQEFKRLQQNAMACKFNWDESAEQYLDMYLGGEQSLVNEICNDKVDA |
| | Em GIgA | MNIVLAASEAFPFCKTGGLADVTGSLAKELSKHGNRVILFLPHYRNINRVASLKVVPGTFLIPIGD RLETASLSYISWGNVLVFFINNTKYFDRPELYRTAAGDYFDNDERFIFFNRAVLESCKFIGYRPDIIH AHDWQAGLLPAYLKTVYKTDAFFTRTRSLFTIHNMAYQGQYPYSTFIKTGFHTVDYVPERFEYYG GISYLKSGIVYADYVNTVSPNYAKEITLDEKMGFGMEGLLRSRQDTFCGILNGLDTGVWDPEHD PLIPYSYESFSPVKGKAACKQFLQNMLGLEVSPSKPLVGIVSRMDYQKGLDLIPGVVNKYKDKVQ FVVVGTGDSGMEKAFMALAKNNVGKVAYVAKVDEELAHRVYAGSDIFLMPSRFEPCGLSQMIS MRYGTVPIVSRVGGLLDTVKGYDGITKYATGFFILEFSETGIERSLDYALKYYQDKRCWGMLIKNG MEKDFSWTKSAQEYQDLYKKIISK |

### Production of starch by recombinant strains using acetic acid

The recombinant *Yarrowia lipolytica* strains were inoculated into a mineral salt medium with acetic acid as the only carbon source (2.73 g/L sodium acetate, 6.75 g/L ammonium sulfate, 13 g/L potassium dihydrogen phosphate, 0.45 g/L magnesium sulfate heptahydrate, 4 mL/L trace element mother solution (3.0 g/L ferrous sulfate heptahydrate, 4.5 g/L zinc sulfate heptahydrate, 4.5 g/L calcium chloride dihydrate, 1 g/L manganese chloride tetrahydrate, 300 mg/L cobalt chloride hexahydrate, 300 mg/L copper sulfate pentahydrate, 400 mg/L sodium molybdate dihydrate, 1 g/L boracic acid, 100 mg/L potassium iodide, 19 g/L disodium ethylene diamine tetraacetic acid dihydrate, 1 mL/L vitamin mother solution (50 mg/L D-biotin, 1.0 g/L D-pantothenic acid hemicalcium salt, 1.0 g/L thiamin-HCl, 1.0 g/L pyridoxin-HCl, 1.0 g/L nicotinic acid, 0.2 g/L 4-aminobenzoic acid, and 25 g/L myo-inositol)), and cells were cultured at 30°C and 220 r/min until the OD600 was about 1.5, centrifuged at 13000 r/min for 2 min, collected, and washed with distilled water twice.

The concentration of the cultured strains was adjusted to 10 OD/mL, and cells were centrifuged and then re-suspended in a lysis solution (20 mM Tris-CI, 500 mM NaCl, pH 7.5) for ultrasonication. The ultrasonication procedure was as follows: ultrasonication for 5 s, pause for 5 s, and the total time was 10 min. 50 µL ultrasonicated solution was boiled at 100°C for 5 min and incubated with 30 U/mL α-amylase and 30 U/mL glucoamylase for a period of time until the starch was completely hydrolyzed into glucose, and then the content of glucose was tested by a glucose determination kit (APPLYGEN). The yield of starch was expressed by the content of glucose. The results were shown in Table 4.

**Table 4. Levels of starch accumulation by fermentation of recombinant Yarrowia lipolytica strains heterogeneously expressing glucose-1-phosphate adenylyltransferase (EC 2.7.7.27) and starch synthase (EC 2.4.1.21)**

| Glucose-1-phosphate adenylyltransferase | Starch synthase | Starch accumulation (shown in glucose equivalent) |
|---|---|---|
| None | None | + |
| CsGIgA | CsGIgC1 | + + + + + |
| EcGIgA | EcGIgC | + + + |
| DdGIgA1 | SeGIgC | + + + |
| KoGIgA | YpGIgC | + + |
| SmGlgA | PIGIgC | + + + |
| YpGIgA | BcGIgC | ++ |
| DdGIgA2 | MtGIgC | + + + |
| PpGIgA | SoGIgC | + + + + |
| EmGIgA | SsGIgC | + + |

| | | |
|---|---|---|
| Note: More + indicates more starch accumulation. | | |

Therefore, the recombinant *Yarrowia lipolytica* strains constructed in this example can effectively produce starch using acetic acid, and the production efficiency of starch can be improved by selecting the sources or specific sequences of the glucose-1-phosphate adenylyltransferase and the starch synthase.

### Example 6: Preparation of starch using Methylobacterium

### Construction of recombinant Methylobacterium

According to sequences information (AAC76454.1) of GlgA (starch synthase, EC 2.4.1.21) and sequences information (AAC76455.1) of GlgC (glucose-1-phosphate adenylyltransferase, EC 2.7.7.27) derived from *Escherichia coli* in GenBank, primers were designed respectively. GlgA and GlgC genes were cloned into a pCM vector by a Gibson assembly method, where both the GlgA and GlgC genes were expressed by separate pMxa promoters to obtain a recombinant plasmid pCM-GlgA-GlgC, and the plasmid was transformed into *Methylobacterium* by chemical transformation to obtain recombinant *Methylobacterium* strain.

### Production of starch by recombinant strains using methanol

The recombinant *Methylobacterium* was inoculated into a medium with methanol as a carbon source (32 g/L methanol, 1 g/L potassium nitrate, 0.717 g/L potassium dihydrogen phosphate, 0.272 g/L disodium hydrogen phosphate, 1 g/L magnesium sulfate, 0.2 g/L calcium chloride, 1 mL/L trace element solution (0.5 g/L ethylene diamine tetraacetic acid, 0.2 g/L ferrous sulfate, 0.01 g/L zinc sulfate, 0.003 g/L manganese chloride, 0.02 g/L cobalt chloride, 0.1 g/L copper sulfate, and 0.003 g/L sodium molybdate₄)), and cultured at 30°C and 220 r/min.

The concentration of the cultured strains was adjusted to 10 OD/mL, and cells were centrifuged and then re-suspended in a lysis solution (20 mM Tris-Cl, 500 mM NaCl, pH 7.5) for ultrasonication. The ultrasonication procedure was as follows: ultrasonication for 5 s, pause for 5 s, and the total time was 10 min. 50 µL ultrasonicated solution was boiled at 100°C for 5 min and incubated with 30 U/mL α-amylase and 30 U/mL glucoamylase for a period of time until the starch was completely hydrolyzed into glucose, and then the content of glucose was tested by a glucose determination kit (APPLYGEN). The yield of starch was expressed by the content of glucose. The results were shown in Table 5.

**Table 5. Levels of starch accumulation by fermentation of recombinant Methylobacterium strains expressing glucose-1-phosphate adenylyltransferase (EC 2.7.7.27) and starch synthase (EC 2.4.1.21)**

| | Starch accumulation (shown in glucose equivalent) |
|---|---|
| Control strain | - |
| Recombinant strain | + + |

| | |
|---|---|
| Note: More + indicates more starch accumulation. | |

In the description of this specification, descriptions with reference to the term such as "an embodiment", "some embodiments", "example", "specific example", or "some examples" mean that specific features, structures, materials, or characteristics described with reference to the embodiment or example are included in at least one embodiment or example of the present application. In this specification, illustrative expressions of the above terms do not refer to the same embodiment or example. Moreover, the described specific features, structures, materials or characteristics can be combined appropriately in one or more embodiments or examples. In addition, without mutual contradiction, those skilled in the art may incorporate and combine different embodiments or examples and features of different embodiments or examples described in this specification.

Although the embodiments of the present application are shown and described above, it can be understood that the foregoing embodiments are examples and cannot be construed as limitations on the present application. Those of ordinary skill in the art can make changes, modifications, substitutions, and variations to the foregoing embodiments within the scope of the present application.

## Claims

1. A method for preparing starch using carbon dioxide, **characterized by** comprising:
(1) providing energy and carbon sources for microbial cells on the basis of carbon dioxide and extracellular non-optical energy; and
(2) generating starch within the microbial cells on the basis of at least one of up-regulated glucose-1-phosphate adenylyltransferase and starch synthase in the microbial cells, wherein
optionally, the extracellular non-optical energy comprises at least one of hydrogen energy and electric energy.

2. The method according to claim 1, **characterized in that** the metabolism of starch is blocked by at least one of glucan 1,4-α-glucosidase and glycogen phosphorylase in a microorganism.

3. The method according to claim 1 or 2, **characterized in that** step (1) further comprises:
enabling the microbial cells to ingest the carbon dioxide as a carbon source and absorb the extracellular non-optical energy; and/or
obtaining a low-carbon compound on the basis of carbon dioxide using the reducing capability of the extracellular non-optical energy, and enabling the microorganism to ingest the low-carbon compound as a carbon source.

4. The method according to claim 3, **characterized in that** the microorganism is at least one of modified yeast and bacteria to be suitable for using the carbon source.

5. The method according to claim 3, **characterized in that** the low-carbon compound contains 1-3 carbon atoms, and
optionally, the low-carbon compound is at least one selected from carbon monoxide, formic acid, methanol, methane, ethanol, formaldehyde, acetaldehyde, acetic acid, propanol, propane, propionaldehyde, acetone, hydroxyacetone, dihydroxyacetone, and glycerol.

6. The method according to claim 3, **characterized in that** the microorganism can transform the low-carbon compound containing 1-3 carbon atoms, and
optionally, the microorganism is at least one selected from the following: *Geobacter, Sporomusa, Methanogens, Acetobacterium, Ralstonia, Clostridium, Pichia pastoris,* hydrogen-oxidizing bacteria, *Cupriavidus necator, Candida boidinii, Hansenula polymorpha, Methylobacter, Methylococcus, Yarrowia lipolytica,* and *Saccharomyces cerevisiae.*

7. The method according to claim 3, **characterized in that** the microorganism uses the carbon dioxide or the low-carbon compound as a main carbon source.

8. The method according to claim 3, **characterized in that** at least one of the starch synthase and the glucose-1-phosphate adenylyltransferase is integrated in a genome of the microbial cells in a form of an exogenous gene, or
in the microbial cells, at least one of the starch synthase and the glucose-1-phosphate adenylyltransferase is contained in a free expression vector,
optionally, the starch synthase and the glucose-1-phosphate adenylyltransferase are independently operably connected with a constitutive promoter or an inducible promoter, and
optionally, the inducible promoter is suitable for improving the transcription level under the induction of the carbon source.

9. The method according to claim 8, **characterized in that** both the starch synthase and the glucose-1-phosphate adenylyltransferase are controlled by the inducible promoters.

10. The method according to claim 8, **characterized in that** the constitutive promoter comprises at least one selected from a ZWF1 glucose-6-phosphate dehydrogenase promoter, a TPI1 triose phosphate isomerase promoter, a GSH1 glutathione synthase promoter, a POR1 mitochondrial porin promoter, a TKL1 transketolase promoter, a PGD1 6-phosphogluconate dehydrogenase promoter, a PGM1 phosphoglycerate mutase promoter, a PK pyruvate kinase promoter, and a GAP glyceraldehyde-3-phosphate dehydrogenase promoter.

11. The method according to claim 8, **characterized in that** the inducible promoter comprises at least one selected from a CAT1 catalase promoter, a TAL1 transaldolase 1 promoter, a TAL2 promoter, an ALD4 acetaldehyde dehydrogenase 4 promoter, a DAK1 dihydroxyacetone kinase promoter, an FDH1 formate dehydrogenase promoter, an ALD acetaldehyde dehydrogenase promoter, a DAS1 dihydroxyacetone synthase 1 promoter, a DAS2 dihydroxyacetone synthase 2 promoter, an AOX1 alcohol oxidase 1 promoter, and an AOX2 alcohol oxidase 2 promoter.

12. The method according to claim 9, **characterized in that** the blocking on the metabolism of starch by at least one of glucan 1,4-α-glucosidase and glycogen phosphorylase is performed by mutating a gene encoding at least one of glucan 1,4-α-glucosidase and glycogen phosphorylase.

13. The method according to claim 12, **characterized in that** the gene encoding the glucan 1,4-α-glucosidase is mutated using SgRNA selected from the following:
SgRNA7: gagtcgataacgatctcctt,
SgRNA10: gttgttgatgtagccgtcta, or
SgRNA 32: ggacgtgatcagggaacatg.

14. The method according to claim 12, **characterized in that** the gene encoding the glycogen phosphorylase is mutated using SgRNA selected from the following:
SgRNA1-509: ggccacctccgactcaatca,
SgRNA6-509: gttaataagagcgttgtcca, or
SgRNA10-1018: gagaagtcaaactcggtggt.

15. A recombinant microorganism for preparing starch using carbon dioxide, **characterized in that** the recombinant microorganism has:
(1) an enzyme system for obtaining energy and carbon sources on the basis of carbon dioxide and extracellular non-optical energy; and
(2) at least one of up-regulated starch synthase and glucose-1-phosphate adenylyltransferase compared with a wild type of the microorganism, wherein
optionally, the extracellular non-optical energy comprises at least one of hydrogen energy and electric energy.

16. The recombinant microorganism according to claim 15, **characterized in that** the recombinant microorganism has at least one of down-regulated glucan 1,4-α-glucosidase and glycogen phosphorylase compared with the wild type of the microorganism.

17. The recombinant microorganism according to claim 15 or 16, **characterized in that** microbial cells are suitable for ingesting the carbon dioxide as a carbon source and absorbing the extracellular non-optical energy; and/or
the microorganism is suitable for ingesting a low-carbon compound as a carbon source, and the low-carbon compound can be obtained on the basis of carbon dioxide using the reducing capability of the extracellular non-optical energy.

18. The recombinant microorganism according to claim 17, **characterized in that** the microorganism is at least one of modified yeast and bacteria to be suitable for using the carbon source.

19. The recombinant microorganism according to claim 17, **characterized in that** the low-carbon compound contains 1-3 carbon atoms, and
optionally, the low-carbon compound is at least one selected from carbon monoxide, formic acid, methanol, methane, ethanol, formaldehyde, acetaldehyde, acetic acid, propanol, propane, propionaldehyde, acetone, hydroxyacetone, dihydroxyacetone, and glycerol.

20. The recombinant microorganism according to claim 17, **characterized in that** the recombinant microorganism can transform the low-carbon compound containing 1-3 carbon atoms, and optionally, a starting strain of the recombinant microorganism is at least one selected from the following:
*Geobacter, Sporomusa, Methanogens, Acetobacterium, Ralstonia, Clostridium, Pichia pastoris,* hydrogen-oxidizing bacteria, *Cupriavidus necator, Candida boidinii, Hansenula polymorpha, Methylobacter, Methylococcus, Yarrowia lipolytica,* and *Saccharomyces cerevisiae.*

21. The recombinant microorganism according to claim 17, **characterized in that** the recombinant microorganism is suitable for using the carbon dioxide or the low-carbon compound as a main carbon source.

22. The recombinant microorganism according to claim 15, **characterized in that** at least one of the starch synthase and the glucose-1-phosphate adenylyltransferase is integrated in a genome of the microbial cells in a form of an exogenous gene, or
in the microbial cells, at least one of the starch synthase and the glucose-1-phosphate adenylyltransferase is contained in a free expression vector.

23. The recombinant microorganism according to claim 22, **characterized in that** the starch synthase and the glucose-1-phosphate adenylyltransferase are independently operably connected with a constitutive promoter or an inducible promoter.

24. The recombinant microorganism according to claim 23, **characterized in that** the inducible promoter is suitable for improving the transcription level under the induction of the carbon source.

25. The recombinant microorganism according to claim 23, **characterized in that** both the starch synthase and the glucose-1-phosphate adenylyltransferase are controlled by the inducible promoters.

26. The recombinant microorganism according to claim 23, **characterized in that** the constitutive promoter comprises at least one selected from a ZWF1 glucose-6-phosphate dehydrogenase promoter, a TPI1 triose phosphate isomerase promoter, a GSH1 glutathione synthase promoter, a POR1 mitochondrial porin promoter, a TKL1 transketolase promoter, a PGD1 6-phosphogluconate dehydrogenase promoter, a PGM1 phosphoglycerate mutase promoter, a PK pyruvate kinase promoter, and a GAP glyceraldehyde-3-phosphate dehydrogenase promoter, and
the inducible promoter comprises at least one selected from a CAT1 catalase promoter, a TAL1 transaldolase 1 promoter, a TAL2 promoter, an ALD4 acetaldehyde dehydrogenase 4 promoter, a DAK1 dihydroxyacetone kinase promoter, an FDH1 formate dehydrogenase promoter, an ALD acetaldehyde dehydrogenase promoter, a DAS1 dihydroxyacetone synthase 1 promoter, a DAS2 dihydroxyacetone synthase 2 promoter, an AOX1 alcohol oxidase 1 promoter, and an AOX2 alcohol oxidase 2 promoter.

27. The recombinant microorganism according to claim 15, **characterized in that** a gene encoding at least one of the 1,4-α-glucosidase and the glycogen phosphorylase carries a mutant to block the metabolism of starch by at least one of the glucan 1,4-α-glucosidase and the glycogen phosphorylase.

28. The recombinant microorganism according to claim 27, **characterized in that** the mutation is performed on the gene encoding the glucan 1,4-α-glucosidase using SgRNA selected from the following:
SgRNA7: gagtcgataacgatctcctt,
SgRNA10: gttgttgatgtagccgtcta, or
SgRNA 32: ggacgtgatcagggaacatg.

29. The recombinant microorganism according to claim 27, **characterized in that** the mutation is performed on the gene encoding the glycogen phosphorylase using SgRNA selected from the following:
SgRNA1-509: ggccacctccgactcaatca,
SgRNA6-509: gttaataagagcgttgtcca, or
SgRNA10-1018: gagaagtcaaactcggtggt.

30. A method for constructing the microorganism according to claim 15-29, **characterized by** comprising the following operation on a starting microorganism:
up-regulating at least one of starch synthase and glucose-1-phosphate adenylyltransferase.

31. The method according to claim 30, **characterized by** further comprising:
down-regulating at least one of glucan 1,4-α-glucosidase and glycogen phosphorylase.

32. The method according to claim 15, **characterized by** comprising modifying a metabolic system of the starting microorganism, so as to have the enzyme system for obtaining energy and carbon sources on the basis of carbon dioxide and extracellular non-optical energy, wherein
optionally, the extracellular non-optical energy comprises at least one of hydrogen energy and electric energy.

33. The method according to claim 31 or 32, **characterized by** modifying the starting microorganism, so that
microbial cells are suitable for ingesting the carbon dioxide as a carbon source and absorbing the extracellular non-optical energy; and/or
the microorganism is suitable for ingesting a low-carbon compound as a carbon source, and the low-carbon compound can be obtained on the basis of carbon dioxide using the reducing capability of the extracellular non-optical energy.

34. The method according to claim 33, **characterized in that** the starting microorganism is at least one of modified yeast and bacteria to be suitable for using the carbon source.

35. The method according to claim 33, **characterized in that** the low-carbon compound contains 1-3 carbon atoms, and
optionally, the low-carbon compound is at least one selected from carbon monoxide, formic acid, methanol, methane, ethanol, formaldehyde, acetaldehyde, acetic acid, propanol, propane, propionaldehyde, acetone, hydroxyacetone, dihydroxyacetone, and glycerol.

36. The method according to claim 17, **characterized in that** the recombinant microorganism can transform the low-carbon compound containing 1-3 carbon atoms, and optionally, the starting microorganism is at least one selected from the following:
*Geobacter, Sporomusa, Methanogens, Acetobacterium, Ralstonia, Clostridium, Pichia pastoris,* hydrogen-oxidizing bacteria, *Cupriavidus necator, Candida boidinii, Hansenula polymorpha, Methylobacter, Methylococcus, Yarrowia lipolytica,* and *Saccharomyces cerevisiae.*

37. The method according to claim 33, **characterized in that** the recombinant microorganism is suitable for using the carbon dioxide or the low-carbon compound as a main carbon source.

38. The method according to claim 30, **characterized in that** at least one of the starch synthase and the glucose-1-phosphate adenylyltransferase is integrated in a genome of the microbial cells in a form of an exogenous gene, or
in the microbial cells, at least one of the starch synthase and the glucose-1-phosphate adenylyltransferase is contained in a free expression vector.

39. The method according to claim 38, **characterized in that** the starch synthase and the glucose-1-phosphate adenylyltransferase are independently operably connected with a constitutive promoter or an inducible promoter.

40. The method according to claim 39, **characterized in that** the inducible promoter is suitable for improving the transcription level under the induction of the carbon source.

41. The method according to claim 39, **characterized in that** both the starch synthase and the glucose-1-phosphate adenylyltransferase are controlled by the inducible promoter.

42. The method according to claim 39, **characterized in that** the constitutive promoter comprises at least one selected from a ZWF1 glucose-6-phosphate dehydrogenase promoter, a TPI1 triose phosphate isomerase promoter, a GSH1 glutathione synthase promoter, a POR1 mitochondrial porin promoter, a TKL1 transketolase promoter, a PGD1 6-phosphogluconate dehydrogenase promoter, a PGM1 phosphoglycerate mutase promoter, a PK pyruvate kinase promoter, and a GAP glyceraldehyde-3-phosphate dehydrogenase promoter, and
the inducible promoter comprises at least one selected from a CAT1 catalase promoter, a TAL1 transaldolase 1 promoter, a TAL2 promoter, an ALD4 acetaldehyde dehydrogenase 4 promoter, a DAK1 dihydroxyacetone kinase promoter, an FDH1 formate dehydrogenase promoter, an ALD acetaldehyde dehydrogenase promoter, a DAS1 dihydroxyacetone synthase 1 promoter, a DAS2 dihydroxyacetone synthase 2 promoter, an AOX1 alcohol oxidase 1 promoter, and an AOX2 alcohol oxidase 2 promoter.

43. The method according to claim 30, **characterized in that** at least one of glucan 1,4-α-glucosidase and glycogen phosphorylase is down-regulated by carrying a mutant in a gene encoding at least one of the 1,4-α-glucosidase and the glycogen phosphorylase to block the metabolism of starch by at least one of the glucan 1,4-α-glucosidase and glycogen phosphorylase.

44. The method according to claim 43, **characterized in that** the mutation is performed on the gene encoding the glucan 1,4-α-glucosidase using SgRNA selected from the following:
SgRNA7: gagtcgataacgatctcctt,
SgRNA10: gttgttgatgtagccgtcta, or
SgRNA 32: ggacgtgatcagggaacatg.

45. The method according to claim 43, **characterized in that** the mutation is performed on the gene encoding the glycogen phosphorylase using SgRNA selected from the following:
SgRNA1-509: ggccacctccgactcaatca,
SgRNA6-509: gttaataagagcgttgtcca, or
SgRNA10-1018: gagaagtcaaactcggtggt.
